# EUROPEAN PATENT APPLICATION

(11) **EP 4 474 016 A1**
(43) Date of publication of application: **11.12.2024**
(21) Application number: 23178054.5
(22) Date of filing: 07.06.2023
(51) Int. Cl.: A61P 35/00

(54) **STRATEGIES TO TARGET EXTRACELLULARLY ACCESSIBLE RAS PROTEIN**

(71) Applicant: Julius-Maximilians-Universität Würzburg, 97070 Würzburg (DE)
(72) Inventor: The designation of the inventor has not yet been filed
(74) Representative: Engelhard, Markus

(57) **Abstract**

The invention is based on the finding of extracellular accessible Ras protein on cancer cells. The invention provides an Antigen Binding Protein (ABP), such as an antibody or a chimeric antigen receptor (CAR), wherein the ABP comprises at least a first antigen binding domain capable of binding to an extracellular Ras antigen. Further provided are isolated nucleic acids and expression constructs for expressing the ABP according to the present invention, as well as recombinant host cells expressing an ABP according to the present invention. The invention also relates to pharmaceutical compositions, pharmaceutical combinations, compounds for use in medicine, and compounds for use in the diagnosis, prevention and/or treatment of cancer. Finally, the present invention pertains to a combination of an ABP, a CAR, an isolated nucleic acid, an expression construct, a recombinant host cell, or a pharmaceutical composition as defined herein, and a chemical compound, such as a cereblon (CRBN) inhibitor, or a polar organic solvent like DMSO, for use in a method of diagnosis, prevention and/or treatment of cancer.

## Description

### FIELD OF THE INVENTION

The invention is based on the finding of extracellular accessible Ras protein on cancer cells. The invention provides an Antigen Binding Protein (ABP), such as an antibody or a chimeric antigen receptor (CAR), wherein the ABP comprises at least a first antigen binding domain capable of binding to an extracellular Ras antigen. Further provided are isolated nucleic acids and expression constructs for expressing the ABP according to the present invention, as well as recombinant host cells expressing an ABP according to the present invention. The invention also relates to pharmaceutical compositions, pharmaceutical combinations, compounds for use in medicine, and compounds for use in the diagnosis, prevention and/or treatment of cancer. Finally, the present invention pertains to a combination of an ABP, a CAR, an isolated nucleic acid, an expression construct, a recombinant host cell, or a pharmaceutical composition as defined herein, and a chemical compound, such as a cereblon (CRBN) inhibitor, or a polar organic solvent like DMSO, for use in a method of diagnosis, prevention and/or treatment of cancer.

### DESCRIPTION

The family of Ras ("Rat sarcoma virus") proteins belong to a class of proteins called small GTPase. Ras proteins are involved in transmitting signals within cells (cellular signal transduction), and are important for regulating cell growth, cell proliferation, and cell differentiation.

There are three Ras genes in the human genome, namely Kirsten rat sarcoma viral oncogene homolog (KRAS), neuroblastoma RAS viral (v-RAS) oncogene homolog (NRAS), and Harvey rat sarcoma viral oncogene homolog (HRAS). For KRAS, two isoforms arise from alternative RNA splicing, namely KRAS4A and KRAS4B. For HRAS, two isoforms arise from an alternate exon resulting in a frameshift and an early stop codon compared to wild type isoform 1. The encoded HRAS isoform 2 has a shorter and distinct C-terminus compared to wild type isoform 1. Thus, there is a total of five different Ras Proteins expressed in human cells.

The amino-terminal catalytic domains of H-Ras, N-Ras and K-Ras are highly conserved (90-100% identical), but the carboxy terminal sequences diverge significantly, carrying the hypervariable domain (HVR). The HVR comprises the anchor sequences, important for trafficking and subcellular localization. Under normal circumstances, the different Ras proteins are attached to the cell membrane from the cytosolic site, which is essential for their activation and down-stream signaling pathways.

Mutations in Ras genes can lead to the production of permanently activated Ras proteins, which can cause unintended and overactive signaling inside the cell, even in the absence of incoming signals. Because these signals result in cell growth and division, overactive Ras signaling can ultimately lead to cancer. Thus, Ras genes are protooncogenic, i.e. mutations in Ras genes can render them oncogenic, and this is causally linked to the development of certain types of cancer. Importantly, the three Ras genes in humans (HRAS, KRAS, and NRAS) are the most common oncogenes in human cancer, and oncogenic mutations that permanently activate Ras are found in 20 to 25% of all human tumors and up to 90% in certain types of cancer (*e.g*., pancreatic cancer, but also lung or colorectal carcinoma are often characterized by a high percentage of oncogenic Ras mutations). Of note, accumulation of constantly active Ras proteins permanently stimulates cell growth.

Specifically, gain-of-function mutations in all three Ras genes are found in 27% (range 0.8%-88%) of all human cancers, with 98% of the mutations at one of the three mutational hotspots - G12, G13 and Q61 - of the Ras protein. The Ras mutation frequency differs for different types of cancer with 1% in low grade glioma, 5.3% in acute myeloid leukemia, 18% in plasma cell myeloma and up to 88% in pancreatic adenocarcinoma.

Mutated Ras is involved in survival and proliferation of malignant cells. Therefore, different strategies are under development to suppress Ras oncogenic signals. Such strategies commonly target upstream proteins, downstream proteins, and/or Ras directly, or by RNA interference, and Ras inhibitors are being studied as a treatment for cancer and other diseases characterized by Ras overexpression. Because Ras proteins are believed to be only intracellular proteins and not expressed on the cell surface, most treatment strategies against the Ras protein focus on compounds able to cross the cell membrane to block Ras or its signaling partners intracellularly.

One example for such a compound under development is Sotorasib, which is a RAS GTPase family inhibitor being developed for the treatment of solid tumors with KRAS mutations, including non-small cell lung cancer (NSCLC) and colorectal cancer. Sotorasib inactivates KRAS by selectively binding to a Cysteine of a specific KRAS mutation ^{[10]}.

Ras must not be mutated in a cancer cell to provide crucial cell signaling, cell proliferation and/ or cell survival benefits. The childhood myelomonocytic leukemia cell line MV-4-11 has no mutation in Ras, but has an FMS-like tyrosine kinase-3 internal tandem duplication (FLT3-ITD), the most frequent mutations found in acute myeloid leukemia (AML). The MV-4-11 cells show a FLT3-ITD-dependent constitutive activation of K-Ras and N-Ras, with the activation of the down-stream Ras/Erk pathway. The Ras activation in these cells is observed primarily at the cell plasma membrane. For solid tumours, non-mutated KRAS is a critical regulator for the metastatic behavior associated with mesenchymal features of breast cancer cells. These data are supporting the view that the mutation status of Ras is not the only important factor for the success of an anti-Ras directed immunotherapy, but rather the dependency of the Ras signaling pathway for the cancer cell.

However, there is so far no treatment available for certain types of cancer, particularly different types of neuroendocrine carcinoma, such as "high-grade poorly differentiated" neuroendocrine carcinoma (NEC-G3) of the gastrointestinal tract. Unfortunately, for NEC-G3 patients, the median survival is usually only a few months. While it has been a long-felt need to develop therapies for these types of tumors, such as NEC-G3 tumors, there was so far no suitable cell line available to assist in therapeutic development.

Wang, et al.^{[1]} generated murine T cells and subsequently isolated T-cell receptors (TCR) highly reactive to the mutated KRAS variants G12V and G12D, and analyzed adoptive transfer of TCR-engineered T cells that recognize mutated KRAS.

The document Douglass, et al.^{[2]} discloses bispecific T-cell engaging antibodies targeting tumor specific mutant RAS neoantigens presented in complex with MHC (e.g. Ras peptides comprising the G12V, Q61H, Q61L, or Q61R mutation). These RAS neoantigens are patient-specific short peptide fragments that are specific for a particular mutation, which are presented on the cell surface in complex with Human leukocyte antigens (HLA). They are the result of mutations during oncogenesis, and allow individualized therapies, i.e. therapies for a small patient group.

EP3310811 B1 discloses bispecific anti-CD3 antibodies having a binding specificity against a second biological molecule, which is a peptide in a HLA-complex. One example of the second biological molecule is a Ras oncogenic neoantigen.

Unfortunately, targeting mutant RAS peptides presented via HLA on the surface of cancer cells with a bispecific diabody has so far only been successful *in vitro* in cell culture experiments, but failed in *in vivo* mouse experiments ⁽²⁾. Maybe due to low expression of RAS peptides via HLA on the surface of cancer cells *in vivo* or due to failure of the T cell recruiting bispecific antibody to for a functional immunologic synapse between the T cells and the tumours cells *in vivo.* Moreover, targeting aberrant RAS peptides in complex with HLA on the cell surface only allows treating few individuals having a particular mutation in a Ras protein and the specific HLA genotype.

The problem to be solved by the present invention is to provide a strategy for treating a broad patient group, independent of the patient HLA genotype, suffering from a disease, such as cancer, for example pancreas carcinoma, colon cancer, breast cancer, neuroendocrine carcinoma, brain cancer, myeloid malignancies, lymphoid malignancies or other type of cancers. It is therefore an object of the present invention to provide a compound that can target cells that are Ras-oncogenic, which are associated with various different Ras mutations, or target cells that have no mutation in the Ras genes, but show a hyperactivation of the Ras signaling complex through other oncogenes. It is a further aim of the present invention to develop an Antigen Binding Protein (ABP), such as an antibody or a chimeric antigen receptor (CAR), capable of binding to an extracellular Ras antigen. It is yet another aim of the present invention to develop T cell-engaging Antigen Binding Proteins (ABPs), such as antibodies, with a Ras binding domain. It is a further aim to provide a use of such ABPs for T-cell mediated immunotherapeutic approaches. Specifically, it is an object of the present invention to provide a use of anti-Ras antibody-based therapies or cell-based therapies like chimeric antigen receptors (CARs) transfected T cells in the prevention and/or treatment of different types of cancer, such as Ras-dependent cancers. It is a further aim to provide a use of such ABPs for diagnostic use, to detect cells aberrantly expressing Ras protein on the cell surface *in vitro* or *in vivo.*

Importantly, the inventor was the first to derive primary tumor cells of a liver metastasis of a NEC-G3 patient and culture these cells in cell culture. From these cells, a stable NEC-G3 cell line was developed, which was used besides primary patient cells for the search of target antigens to develop novel antibody-based immunotherapies for cancer, such as NEC-G3 carcinoma. Thereby, the inventor was the first to discover that Ras protein is aberrantly expressed to a low amount on the cell surface of cancer cells. Importantly, the finding of aberrantly expressed extracellular accessible Ras protein on cancer cells enables the possibility to target Ras mutated cancer cells, and/or Ras wild type cancer cells but with activation of the Ras signaling pathways by other mutations, with an antibody and/or Ras binding format from the outside of a cell.

### BRIEF DESCRIPTION OF THE INVENTION

Generally, and by way of brief description, the main aspects of the present invention can be described as follows:
In a first aspect, the invention pertains to an Antigen Binding Protein (ABP).
In a second aspect, the invention pertains to a Chimeric Antigen Receptor (CAR).
In a third aspect, the invention pertains to an isolated nucleic acid comprising a sequence encoding for an ABP according to the present invention, or for a CAR according to the present invention.
In a fourth aspect, the invention pertains to an expression construct for expressing the ABP according to the present invention, or a CAR according to the present invention, in a (host) cell, preferably further comprising promoter and/or terminator sequences.
In a fifth aspect, the invention pertains to a recombinant host cell expressing an ABP according to the present invention or a CAR according to the present invention, or comprising an isolated nucleic acid according to the present invention, or an expression construct according to the present invention.
In a sixth aspect, the invention pertains to a pharmaceutical composition comprising: (i) an ABP according to the present invention, or (ii) a CAR according to the present invention, or (iii) an isolated nucleic acid according to the present invention, or (iv) an expression construct according to the present invention, or (v) a recombinant host cell according to the present invention, and a pharmaceutically acceptable carrier, stabilizer and/or excipient.
In a seventh aspect, the invention pertains to a pharmaceutical combination comprising:
   (i) an ABP, a CAR, an isolated nucleic acid, an expression construct, a recombinant host cell, or a pharmaceutical composition according to the present invention, and
   (ii) a chemical compound, wherein the chemical compound is an organic solvent or an inhibitor increasing the expression of Ras protein on the extracellular site of the plasma membrane, preferably wherein the chemical compound is a cereblon (CRBN) inhibitor, or a polar organic solvent like Dimethylsulfoxid (DMSO).
In an eighth aspect, the invention pertains to a compound for use in medicine.
In a ninth aspect, the invention pertains to a compound for use in the diagnosis, prevention and/or treatment of a proliferative disorder, such as cancer, or for use in adoptive, target-cell specific immunotherapy.
In a tenth aspect, the invention pertains to a combination of an ABP, a CAR, an isolated nucleic acid, an expression construct, a recombinant host cell, or a pharmaceutical composition according to the present invention, and a chemical compound, wherein the chemical compound is an organic solvent or an inhibitor increasing the expression of Ras protein on the extracellular site of the plasma membrane, preferably wherein the chemical compound is a cereblon (CRBN) inhibitor, or a polar organic solvent like Dimethylsulfoxid (DMSO), for use in a method of diagnosis, prevention and/or treatment of a proliferative disorder, such as cancer.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to an Antigen Binding Protein (ABP), wherein the ABP comprises at least a first antigen binding domain capable of binding to an extracellular Ras antigen, provided that said extracellular Ras antigen is not part of a human leukocyte antigen (HLA)-peptide complex presented on the cell surface by the major histocompatibility complex (MHC), and wherein said ABP optionally comprises one or more additional antigen binding domain(s) that is capable of binding to antigen(s) present on a mammalian T-cell, preferably a human cluster of differentiation 3 (CD3) antigen or a human T cell receptor (TCR) or mammalian natural killer (NK) cells.

According to the present invention, the term "capable of" binding to shall refer to a binding domain that is able to bind to a certain antigen, or is specific for a particular antigen, *i.e.* is specifically binding to said antigen. Thus, the present invention pertains to an ABP, wherein the ABP comprises at least a first antigen binding domain specifically binding to an extracellular Ras antigen, provided that said extracellular Ras antigen is not part of a HLA-peptide complex presented on the cell surface by MHC, and wherein said ABP optionally comprises one or more additional antigen binding domain(s) that specifically bind(s) to antigen(s) present on a mammalian T-cell, preferably a human cluster of differentiation 3 (CD3) antigen or a human T cell receptor (TCR).

Moreover, the term binding domain "capable of binding" shall further refer to a binding domain that is specific for a particular antigen. As such, the present invention pertains to an ABP, wherein the ABP comprises at least a first antigen binding domain that is specific for an extracellular Ras antigen, provided that said extracellular Ras antigen is not part of a HLA-peptide complex presented on the cell surface by MHC, and wherein said ABP optionally comprises one or more additional antigen binding domain(s) that is specific for antigen(s) present on a mammalian T-cell, preferably a human cluster of differentiation 3 (CD3) antigen or a human T cell receptor (TCR).

The present invention thus also relates to an ABP, wherein the ABP comprises at least a first antigen binding domain capable of binding to an extracellular Ras antigen, provided that said extracellular Ras antigen is not part of a HLA-peptide complex presented on the cell surface by MHC, and wherein said ABP comprises at least a second antigen binding domain that is capable of binding to antigens present on a mammalian T-cell, preferably a human cluster of differentiation 3 (CD3) antigen.

Thus, the present invention relates to an ABP having a binding domain for Ras, which mediates tumor specificity. Moreover, the ABP of the present invention preferably also has a binding domain for CD3, which enables the binding to T cells. Most preferably, the present invention relates to an ABP having a binding domain for Ras and a binding domain for CD3. In a particularly preferred example, the present invention relates to a bispecific antibody having a binding domain for Ras and a binding domain for CD3.

The inventor found extracellular accessible Ras protein on cancer cells. The inventor then developed the bi-specific ABPs of the present invention that are able to bind to Ras proteins on the cell surface of cancer cells and CD3 positive T cells, and, thereby, eliminate cancerous cells via a T cell-induced immune response.

The ABP according to the present invention binds to an epitope displayed by one or more extracellular domain(s) of a Ras protein. Importantly, extracellular accessible Ras protein provides a multitude of epitopes including an epitope covering a part of the Ras wildtype sequence. Thus, the ABPs of the present invention can target various cells that aberrantly express mutant Ras and/or wildtype Ras proteins. Therefore, the ABPs of the present invention are highly beneficial over ABPs of the prior art, which target patient-specific RAS neoantigens in complex with MHC. Such RAS neoantigens are the result of mutations during oncogenesis, and allow individualized therapies, *i.e.* therapies for a very small patient group because such neoantigens are specific for a certain mutation.

Importantly, the ABPs of the present invention target extracellular accessible Ras protein that differs largely in its tertiary structure from Ras epitopes presented by short peptide fragments in complex with HLAs. Therefore, the ABPs of the present invention differ from ABPs targeting Ras neoantigenes in complex with HLAs. Importantly, the ABPs of the present invention allow targeting extracellular accessible Ras protein in aggressive tumors on Ras mutated cancer cells from the cell outside.

In one preferred embodiment, the ABP of the present invention can recruit immune cells like natural killer (NK) cells or antigen presenting cells (like dendritic cells).

In a preferred embodiment, the extracellular Ras antigen is selected from a Kirsten rat sarcoma viral oncogene homolog (KRAS), such as KRAS4A or KRAS4B, a neuroblastoma RAS viral (v-RAS) oncogene homolog (NRAS), and a Harvey rat sarcoma viral oncogene homolog (HRAS). Of note, the two KRAS isoforms KRAS4A and KRAS4B arise from alternative RNA splicing. For HRAS, two isoforms arise from an alternate exon resulting in a frameshift and an early stop codon compared to wild type isoform 1. The encoded HRAS isoform 2 has a shorter and distinct C-terminus compared to wild type isoform 1. Thus, there is a total of five different Ras Proteins expressed in human cells, and the extracellular Ras antigen according to the present invention can be any of these.

The present invention further relates to an ABP, wherein the ABP comprises at least a first antigen binding domain capable of binding to an extracellular Ras antigen, a paralogue, an orthologue or other variant thereof.

In another preferred embodiment, which can be combined with any and all other specifically preferred embodiments and aspects of the present invention, the first antigen binding domain of the ABP according to the present invention is capable of binding to an extracellular human Ras antigen.

Particularly preferred is that the extracellular Ras antigen comprises, preferably consists of, the amino acid sequence according to any one of SEQ ID NOs: 1, 3, 5, 7, or 9, or comprises, preferably consists of, a sequence having at least 75% sequence identity, preferably at least 80%, more preferably at least 90%, and most preferably at least 95% sequence identity with any one of SEQ ID NOs: 1, 3, 5, 7, or 9.

According to one preferred embodiment, the present invention thus pertains to an ABP, wherein the ABP comprises at least a first antigen binding domain specifically binding to an extracellular Ras antigen according to any one of SEQ ID NOs: 1, 3, 5, 7, or 9.

In one embodiment, the extracellular Ras antigen is a mutant Ras antigen according to any one of SEQ ID NOs: 11 to 19, or 77 to 119. In one preferred embodiment, the targeted protein is a mutant Ras protein expressed on the extracellular site of a cancer cell. Thus, an ABP of the present invention can target Ras mutated oncogenic cells.

In another preferred embodiment, the cells targeted by the ABP of the present invention are malignant cancer cells and/or Ras mutated cells.

In another preferred embodiment, the malignant cells targeted by the ABP of the present invention have no mutation in Ras, but show Ras cellular signaling dependency. Thus, Ras is important for the tumour cell survival and proliferation.

In a further embodiment, the ABP according to the present invention binds to an extracellular accessible Ras antigen with an EC₅₀ higher than 0.01 nM, preferably higher than 0.1 nM, even more preferably higher than 1 nM, even more preferably higher than 10 nM, even more preferably higher than 20 nM, and most preferably higher than 30 nM.

In a further preferred embodiment, the ABP enhances a cell-mediated immune response, such as the immune response mediated by an activated cytotoxic T-cell (CTL) to a mammalian cell expressing said extracellular accessible Ras antigen.

In a specific embodiment, the one or more additional antigen binding domain(s) of the ABP enhances a cell-mediated immune response, such as that mediated by an activated cytotoxic T-cell (CTL), to a mammalian cell expressing said extracellular Ras antigen.

Thus, the one or more additional antigen binding domain(s) of the ABP that is capable of binding to antigen(s) present on a mammalian T-cell, preferably the human cluster of differentiation 3 (CD3) antigen or a human T cell receptor (TCR), increases the activity of immune cells, such as T-cells.

In one specific embodiment, the specific T cell response is induced by a specific anti-CD3 binding of the at least one second antigen binding domain of the ABP according to the present invention to antigens present on a human cluster of differentiation 3 (CD3) antigen.

In a preferred embodiment, the antigen binding domain(s) that is capable of binding to antigen(s) present on a mammalian T-cell, preferably a human cluster of differentiation 3 (CD3) antigen, comprises an amino acid sequence according to SEQ ID No: 48 or 49.

In another specific embodiment, the one or more additional antigen binding domain(s) of the ABP enhances a cell-mediated immune response, such as that mediated by an activated natural killer (NK) cell, to a mammalian cell expressing said extracellular Ras antigen.

In another specific embodiment, the one or more additional antigen binding domain(s) of the ABP enhances a cell-mediated immune response, such as that mediated by an activated natural killer T cell (NKT cell), to a mammalian cell expressing said extracellular Ras antigen.

In another specific embodiment, the one or more additional antigen binding domain(s) of the ABP enhances a cell-mediated immune response, such as that mediated by an activated regulatory T cell (TReg), to a mammalian cell expressing said extracellular Ras antigen.

In another specific embodiment, the one or more additional antigen binding domain(s) of the ABP enhances a cell-mediated immune response, such as that mediated by an activated antigen-presenting cell (APC) or accessory cell, to a mammalian cell expressing said extracellular Ras antigen.

In a further preferred embodiment, the antigen binding domain capable of binding to an extracellular Ras antigen, provided that said extracellular Ras antigen is not part of a human leukocyte antigen (HLA)-peptide complex presented on the cell surface by the major histocompatibility complex (MHC), comprises an amino sequence according to any one of SEQ ID Nos: 50 to 61.

In one embodiment, the anti CD3 binder of the present invention was derived from the diL2K clone. In another embodiment, the anti CD3 binder of the present invention was derived from the UCHT1 clone. The anti-CD3 antibodies according to the present invention are herein sometimes referred to as diL2K and/or UCHT1. In one embodiment, the anti-CD3 antibodies diL2K and/or UCHT1 bind to the epsilon chain of the CD3 receptor complex.

In a further embodiment, the ABP of the present invention increases type-I cytokine secretion by said immune cells, for example T-cells or CTLs, such as one or more cytokines independently selected from the list consisting of: IFN-gamma, IL-2 and TNF-alpha.

Another embodiment relates to the ABP according to the present invention, wherein said ABP is selected from the group consisting of: an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, a hemibody antibody, a single-chain KeyLock-antibody, a diabody, a single chain diabody, a variable domain of the antibody heavy chain or antibody light chain, a multispecific antibody, a chimeric antigen receptor (CAR), and a fragment of an antibody, such as a fragment of a monoclonal antibody, for example a single chain Fv (scFv), (scFv)2, a Fv, a disulfide linked Fv, Fab, Fab', F(ab')2 or a scFv-Fc, preferably wherein said ABP is a bispecific antibody or a CAR. The ABP can also be selected from the group of alternative protein binders including monobodies (derived from fibronectin type III), anticalins (derived from lipocalins), affibodies (derived from immunoglobulin-binding protein A) or DARPins (Designed Ankyrin Repeat Proteins). These alternative binders usually have a "constant" scaffold and a "variable" site for target antigen binding. Many types of alternative scaffolds are based on proteins with repeating motifs like leucine-rich repeats (LRRs), ankyrin repeats (ARs), Armadillo repeats (Arms), and tetratricopeptide repeats (TPRs).

The invention also includes novel dual antigen restrictedABP constructs, such as antibodies termed hemibodies, for a targeted immunotherapy of patients with cancer.

In one embodiment, the ABP comprises a heavy chain immunoglobulin constant domain selected from the group consisting of:
a human IgM constant domain,
a human IgG1 constant domain,
a human IgG2 constant domain,
a human IgG3 constant domain,
a human IgG4 constant domain,
a human IgE constant domain,
a human IgA constant domain,
and human IgD constant domain,
an IgG constant domain variant with one or more mutations altering binding strength to Fc neonatal receptor, Fc gamma receptors, or C1q.

A further embodiment pertains to the ABP according to the present invention, wherein one, preferably two, heavy chain variable domain(s) and one, preferably two, light chain variable domain(s), each comprise an antibody framework having at least a portion of a human antibody consensus framework sequence.

In one embodiment, the ABP comprises two different heavy chain immunoglobulin constant domains, wherein the first heavy chain has a specific mutation (knop mutation), and the pairing second heavy chain has a corresponding mutation (hole mutation), allowing to form a heterodimer (knop into hole).

In a preferred embodiment, the ABP is a heterodimer comprising two different heavy chain immunoglobulin constant domains, wherein the first heavy chain comprises a first mutation (such as a knop mutation), and the pairing second heavy chain comprises a corresponding second mutation (such as a hole mutation), wherein the first mutation and the second mutation allow to form a heterodimer (knop into hole), optionally wherein the first heavy chain comprises an amino acid sequence according to SEQ ID No 42 and the second heavy chain comprises an amino acid sequence according to SEQ ID No 43.

In a particularly preferred embodiment, the ABP is a bispecific antibody, and comprises at least one additional antigen binding domain that binds to a human cluster of differentiation 3 (CD3) antigen. Accordingly, the present invention provides T cell-engaging antibodies with a Ras binding domain. Importantly, the antibodies of the present invention are thereby able to redirect cytotoxic T cells against tumor cells.

In another particularly preferred embodiment, the ABP is an antibody with a toxic payload directly labeled to it. These Antibody-Drug Conjugates (ADCs) can carry a toxic payload like ozogamicin/calicheamicin, Vedotin (Monomethylauristatin E), Maytansinoide, camptothecin, auristatin or Tesirin (SG-3199). The toxic payload can also be a radioactive label like Yttrium-90, Iodine-131, Samarium-153, Lutetium-177, Astatine-211, Lead-212/bismuth-212, Radium-223, Actinium-225 or Thorium-227.

In a further embodiment, the ABP is used to carry a nucleic acid, such as an RNA molecule, like an siRNA molecule, into RAS expressing cells.

In another particularly preferred embodiment, the ABP is part of an oncolytic virus, for a virus-mediated immune response, such as that mediated by DNA or RNA viruses, to a mammalian cell expressing said extracellular Ras antigen.

Another preferred embodiment relates to a fragment of an ABP according to the present invention, such as a fragment of a bispecific antibody that comprises at least a first antigen binding domain capable of binding to an extracellular Ras antigen, provided that said extracellular Ras antigen is not part of a HLA-peptide complex presented on the cell surface by MHC, and comprises at least one additional antigen binding domain that binds to a human cluster of differentiation 3 (CD3) antigen.

In a further embodiment, said ABP according to the present invention is isolated and/or substantially pure.

Yet another embodiment relates to the ABP according to the present invention, wherein said ABP is not cell membrane permeable.

A further embodiment relates to the ABP according to the present invention, wherein said ABP comprises an effector group.

In one embodiment, the ABP according to the present invention is labelled.

Another embodiment pertains to the ABP according to the present invention, wherein said ABP is Fc receptor binding attenuated.

A further embodiment pertains to the ABP according to the present invention, wherein the ABP comprises at least a first antigen binding domain capable of binding to an extracellular Ras antigen, provided that said extracellular Ras antigen is not part of a HLA-peptide complex presented on the cell surface by MHC, and wherein said ABP optionally comprises one or more additional antigen binding domain(s) that is capable of binding to antigen(s) present on a mammalian T-cell, preferably a human cluster of differentiation 3 (CD3) antigen or a human T cell receptor (TCR),
comprising:
(i) a heavy chain variable domain comprising the CDRH1 region set forth in SEQ ID NO: 50 and/or 53, the CDRH2 region set forth in SEQ ID NO: 51 and/or 54, and the CDRH3 region set forth in SEQ ID NO: 52 and/or 55, or wherein in each case independently the CDRH1, CDRH2 and/or CDRH3 comprise a sequence having no more than three or two, preferably no more than one amino acid substitution(s), deletion(s) or insertion(s) compared to SEQ ID NO: 50 and/or 53, SEQ ID NO: 51 and/or 54, or SEQ ID NO: 52 and/or 55, respectively; or comprising a CDRH1, CDRH2 or CDRH3 sequence having at least 75% sequence identity, preferably at least 80% sequence identity, more preferably at least 90% sequence identity, and most preferably at last 95% sequence identity with SEQ ID NO: 50 and/or 53, SEQ ID NO: 51 and/or 54, or SEQ ID NO: 52 and/or 55; and
(ii) a light chain variable domain comprising the CDRL1 region set forth in SEQ ID NO:56 and/or 59, the CDRL2 region set forth in SEQ ID NO: 57 and/or 60, and the CDRL3 region set forth in SEQ ID NO: 58 and/or 61 or wherein in each case independently CDRL1, CDRL2 and/or CDRL3 comprise a sequence having no more than three or two, preferably no more than one amino acid substitution(s), deletion(s) or insertion(s) compared to SEQ ID NO: 56 and/or 59, SEQ ID NO: 57 and/or 60, or SEQ ID NO: 58 and/or 61, respectively; or comprising a CDRL1, CDRL2 or CDRL3 sequence having at least 75% sequence identity, preferably at least 80% sequence identity, more preferably at least 90% sequence identity, and most preferably at last 95% sequence identity with SEQ ID NO: 56 and/or 59, SEQ ID NO: 57 and/or 60, or SEQ ID NO: 58 and/or 61.

A further embodiment pertains to the ABP according to the present invention, wherein the heavy chain variable region comprises the amino acid sequence having a sequence identity of at least 70%, preferably at least 80%, more preferably at least 90%, and most preferably at last 95% sequence identity, to an amino acid sequence selected from SEQ ID NO: 62 and/or 64, or, in each case independently, optionally with no more than ten, nine, eight, seven, six, five, four, preferably no more than three, two or one, amino acid substitution(s), insertion(s) or deletion(s) compared to these sequences; and/or wherein the light chain variable region comprises the amino acid sequence having a sequence identity of at least 70%, preferably at least 80%, more preferably at least 90%, and most preferably at last 95% sequence identity, to the amino acid sequence selected from SEQ ID NO: 63 and/or 65, or, in each case independently, optionally with no more than ten, nine, eight, seven, six, five, four, preferably no more than three, two or one, amino acid substitution(s), insertion(s) or deletion(s) compared to these sequences. In this embodiment, the heavy chain variable region and the light chain variable region is referring to the anti-CD3 binding domain.

Yet another embodiment pertains to the ABP according to the present invention, comprising at least one antibody heavy chain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NO: 66, 68, or 70; and/or comprising at least one antibody light chain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to a sequence selected from selected from SEQ ID NO: 67, 69, or 71. In this embodiment, the heavy chain variable region and the light chain variable region is referring to the anti-Ras binding domain.

Yet another embodiment pertains to the ABP according to the present invention, comprising a single-domain antibody (sdAb), also known as a nanobody, with a single monomeric variable antibody binding domain having an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from SEQ ID NO: 25, and 26. In one embodiment, the PCC06 expression construct used in the present invention is a V_{H}H single domain antibody.

Yet another embodiment pertains to the ABP according to the present invention, comprising a human protein or a part of a human protein with a known binding site to a Ras protein, wherein the human protein is binding to Ras in its active and/or inactive state, preferably wherein the binding site is a direct binding site. In one embodiment, the ABP according to the present invention comprises a part of a human protein with a known binding site to a Ras protein, wherein only this part of the protein is important for Ras binding.

In one embodiment, the part of a human protein binding to a Ras protein has an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from any one of SEQ ID NOs: 23, 24, 27, 28, 29, 30, and 31. In one embodiment, these proteins are referred to as "alternative Ras binders".

In one embodiment, the ABP according to the present invention comprises an amino acid sequence with at least 80% sequence identity to, or having no more than twenty, fifteen, ten, nine, eight, seven, six, four, preferably three or two, more preferably no more than one amino acid substitution(s), deletion(s) or insertion(s) compared to, a sequence selected from selected from any one of SEQ ID NOs: 23, 24, 27, 28, 29, 30, and 31.

In the second aspect, the invention pertains to a chimeric antigen receptor (CAR), comprising from N-terminus to C-terminus:
(a) an extracellular domain comprising at least an antigen binding domain capable of binding to an extracellular accessible Ras antigen, provided that said extracellular Ras antigen is not part of a HLA-peptide complex presented on the cell surface by MHC, wherein the antigen binding domain comprises the amino acid sequence having a sequence identity of at least 70%, preferably at least 80%, more preferably at least 90%, and most preferably at least 95% sequence identity, to an amino acid sequence selected from SEQ ID NO: 20, 21, 22, 23, 24, 25, 26, 27, 28, 29, 30, 31, 66, 67, 68, 69, 70, or 71 or, in each case independently, optionally with no more than ten, nine, eight, seven, six, five, four, preferably no more than three, two or one, amino acid substitution(s), insertion(s) or deletion(s) compared to these sequences;
(b) an extracellular hinge domain,
(c) a transmembrane domain, and
(d) a cytoplasmic domain,
optionally wherein the extracellular hinge domain, the transmembrane domain and the cytoplasmic domain comprise the amino acid sequence having a sequence identity of at least 70%, preferably at least 80%, more preferably at least 90%, and most preferably at last 95% sequence identity, to an amino acid sequence selected from SEQ ID NO: 72 or, in each case independently, optionally with no more than ten, nine, eight, seven, six, five, four, preferably no more than three, two or one, amino acid substitution(s), insertion(s) or deletion(s) compared to these sequences.

In a preferred embodiment, the CAR according to the present invention specifically binds to an extracellular Ras antigen according to the present invention.

In another embodiment, the CAR molecule according to the present invention is a CAR molecule of the first, second, third, fourth, fifth or next generation, optionally comprising one additional antigen binding domain, such as an scFv antigen binding domain, VHH, DARPIN, or variable NAR-domain.

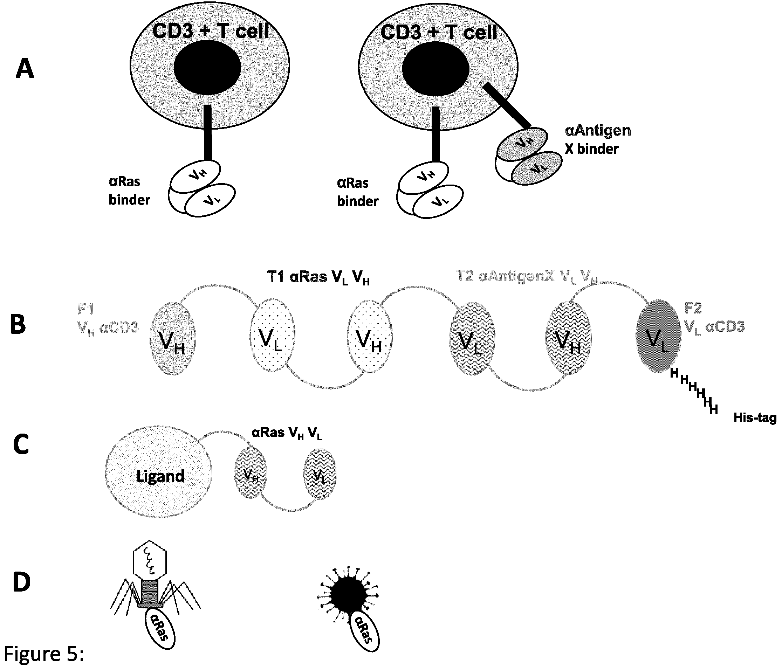

In one embodiment, the CAR according to the present invention is a dual-antigen CAR. One example of such a dual-antigen CAR is shown in Figure 5A, right side.

In one embodiment, when the CAR molecule according to the second aspect of the present invention is expressed in a T cell, such as a CD3+ T cell or a CD8+ T cell, the T cell demonstrates activity against at least 2 different cancer types, at least 3 different cancer types, at least 4 different cancer types, at least 5 different cancer types, at least 6 different cancer types, at least 7 different cancer types, at least 8 different cancer types, at least 9 different cancer types, or at least 10 different cancer types.

Importantly, the novel anti-Ras directed immunotherapy of the present invention can be used for cell-based therapies like chimeric antigen receptors (CARs) transfected T cells for treating different types of Ras-dependent cancers. In one embodiment, CAR-T cells have activity against Ras tumor antigens.

In the third aspect, the invention pertains to an isolated nucleic acid comprising a sequence encoding for an ABP according to the present invention, or for a CAR according to the present invention.

According to a further embodiment, said isolated nucleic acid comprises a sequence encoding for an antigen binding fragment or a monomer, such as a heavy or light chain, of an ABP according to the present invention, or for a CAR according to the present invention.

In one embodiment, the isolated amino acid according to the third aspect of the present invention relates to the amino acid sequence of SEQ ID NO. 72 or their complementary sequences or sequences that have at least 95 % sequence identity with said sequence.

In the fourth aspect, the invention pertains to an expression construct for expressing the ABP according to the present invention, or a CAR according to the present invention, in a (host) cell, preferably further comprising promoter and/or terminator sequences.

According to a further embodiment, said expression construct is for expressing an antigen binding fragment or a monomer, such as a heavy or light chain, of an ABP according to the present invention, or for a CAR according to the present invention.

In one embodiment, the ABP according to the present invention, or the CAR according to the present invention, have protein linkers connecting the different Ras binding domains, connecting the Ras binding domains with the antibody constant domains, connecting the Ras binding domain with the CAR hinge or transmembrane domain, or connecting the detection and/or purification tags with the ABP.

In yet another embodiment, the protein linker comprises an amino acid sequence having a sequence identity of at least 70%, preferably at least 80%, more preferably at least 90%, and most preferably at last 95% sequence identity, to an amino acid sequence selected from SEQ ID NO: 34, 35, 36, 37, 38, 39, 40, or 41, or, in each case independently, optionally with no more than ten, nine, eight, seven, six, five, four, preferably no more than three, two or one, amino acid substitution(s), insertion(s) or deletion(s) compared to these sequences.

Four exemplary protein linkers between the Ig constant domain and Ras binders have an amino acid sequence selected from:
SEQ ID NO 36: GA
SEQ ID NO 37: AS
SEQ ID NO 38: GS
SEQ ID NO 39: GG

Of note, a protein linker comprising the amino acid sequence of SEQ ID NO 38 (GS) can also be a protein linker between a His tag and Ras binders.

In one embodiment, the ABP according to the present invention, has one, two, or more protein tags for detection and/or purification.

In a further embodiment, the protein tag is a His tag and/or a CL7 tag.

In yet another embodiment, the protein tag comprises an amino acid sequence having a sequence identity of at least 70%, preferably at least 80%, more preferably at least 90%, and most preferably at last 95% sequence identity, to an amino acid sequence selected from SEQ ID NO: 32, 33, 45, 46 or, in each case independently, optionally with no more than ten, nine, eight, seven, six, five, four, preferably no more than three, two or one, amino acid substitution(s), insertion(s) or deletion(s) compared to these sequences.

In one embodiment, the protein tag is a protein tag for detection and/or purification, wherein the protein tag has a specific proteolytic cleavage site to be cut off in the production and purification process from the final ABP.

In another embodiment, the protein tag for detection and/or purification comprises an amino acid sequence having a sequence identity of at least 70%, preferably at least 80%, more preferably at least 90%, and most preferably at last 95% sequence identity, to an amino acid sequence selected from SEQ ID NO: 47 or, in each case independently, optionally with no more than ten, nine, eight, seven, six, five, four, preferably no more than three, two or one, amino acid substitution(s), insertion(s) or deletion(s) compared to these sequences.

In one embodiment, the ABP according to the present invention, has two tags.

In another embodiment, the ABP according to the present invention, has a His tag and a CL7 tag.

In yet another embodiment, the expression construct encoding for the ABP according to the present invention, has a His tag and a CL7 tag.

In one embodiment, the CL7 tag is cleaved during the final purification of the ABP according to the present invention, and one, two, three, four, five, six, seven, eight, nine, or ten amino acids remain on the N-terminus on the ABP according to the present invention. In a preferred embodiment, the CL7 tag is cleaved during the final purification of the ABP according to the present invention, and two amino acids remain on the N-terminus on the ABP according to the present invention.

In yet another embodiment, the His tag of the expression construct is also present in the ABP according to the present invention, preferably the antibody according to the present invention. The His tag can be used for the detection of the ABP according to the present invention in serum or tissue.

In one embodiment, the ABP according to the present invention, or the CAR according to the present invention, have a protein secretion leader to express the CAR on the cell surface and/or for extracellular secretion.

In another embodiment, the protein secretion leader has an amino acid sequence having a sequence identity of at least 70%, preferably at least 80%, more preferably at least 90%, and most preferably at last 95% sequence identity, to an amino acid sequence selected from SEQ ID NO: 44 or, in each case independently, optionally with no more than ten, nine, eight, seven, six, five, four, preferably no more than three, two or one, amino acid substitution(s), insertion(s) or deletion(s) compared to these sequences.

The present invention further pertains to a nucleic acid construct (NAC) comprising an isolated nucleic acid according to the present invention and one or more additional sequence features permitting the expression of the encoded ABP, or a component of said ABP (such as an antibody heavy chain or light chain) in a (host) cell.

In the fifth aspect, the invention pertains to a recombinant host cell expressing an ABP according to the present invention or a CAR according to the present invention, or comprising an isolated nucleic acid according to the present invention, or an expression construct according to the present invention, which is preferably selected from effector cells of the immune system, such as lymphocytes, for example CD8 positive cytotoxic lymphocytes, CD4 positive T cells, T helper cells, or Th17 T cells, natural killer (NK) cells, natural killer T (NKT) cells, dendritic cells, killer dendritic cells, B cells, γδ T cells, and a lymphocyte preparation containing NK cells and NKT mast cells. Thus, the present invention further pertains to a host cell transformed with an isolated nucleic acid according to the present invention, or an expression construct according to the present invention.

In a preferred embodiment, the recombinant host cell expresses an ABP capable of binding a Ras antigen, wherein the Ras antigen comprises, preferably consists of, the amino acid sequence according to any one of SEQ ID NOs: 1, 3, 5, 7, or 9, or comprises, preferably consists of, a sequence having at least 75% sequence identity, preferably at least 80%, more preferably at least 90%, and most preferably at least 95% sequence identity with any one of SEQ ID NOs: 1, 3, 5, 7, or 9.

In one example, said recombinant host cell is a Chinese Hamster Overay (CHO) cell, a Human Embryonic Kidney (HEK) cell, a Pichia pastoris cell, a Saccharomyces cerevisiae cell, a Hansenula polymorpha cell, a Schizosaccharomyces pombe cell, a Leishmania tarentolae cell, a Spodoptera frugiperda cell, or a Trichopulsia ni High Five cell.

In one example, said recombinant host is a prokaryote cell, is an Escherichia coli cell, a Caulobacter crescentus cell, a Lactobacillus zeae cell, a Lactococcus lactis cell, a Bacillus brevis cell, a Bacillus subtilis cell, a Bacillus megaterium cell, a Caulobacter crescentus cell, or a Corynebacterium species cell like a Corynebacterium glutamicum cell.

A further embodiment pertains to a therapeutically effective amount of the above recombinant host cell according to the fifth aspect of the present invention, for use in the treatment of a proliferative disorder, such as cancer, and/or for use in adoptive, target-cell specific immunotherapy, in a subject in need thereof. Preferably, the cancer is selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

Yet another embodiment of the present invention pertains to an engineered immune cell (genetically modified cell) expressing the ABP or CAR according to the present invention, wherein preferably the engineered immune cell is selected from the group consisting of a cytotoxic T cell, a helper T cell, a natural killer T cell, a γδT cell, and a NK cell. In a preferred embodiment, the cytotoxic T cell is a CD4+ T cell, a CD8+ T cell, a gamma delta T cell, or a natural killer cell.

In the sixth aspect, the invention pertains to a pharmaceutical composition comprising: (i) an ABP according to the present invention, or (ii) a CAR according to the present invention, or (iii) an isolated nucleic acid according to the present invention, or (iv) an expression construct according to the present invention, or (v) a recombinant host cell according to the present invention, and a pharmaceutically acceptable carrier, stabilizer and/or excipient.

In the seventh aspect, the invention pertains to a pharmaceutical combination comprising:
(i) an ABP according to the present invention, a CAR according to the present invention, an isolated nucleic acid according to the present invention, an expression construct according to the present invention, a recombinant host cell according to the present invention, or a pharmaceutical composition according to the present invention, and
(ii) a chemical compound, wherein the chemical compound is an organic solvent or an inhibitor increasing the expression of Ras protein on the extracellular site of the plasma membrane, preferably wherein the chemical compound is a cereblon (CRBN) inhibitor, or a polar organic solvent like DMSO.

The correct expression of folded and functional receptors is crucial for cell signaling, cell migration, cell proliferation and cell survival. For malignant cells, with gain and/or loss of function mutations altering the cells signaling cascades, the blockade/inhibition of proteins responsible for protein production, processing and/or transport can cause aberrant expression of proteins at the plasma membrane. Of note, the immunomodulatory drug Lenalidomide is inhibiting the protein cereblon (CRBN). In Multiple Myeloma cells, treated with Lenalidomide, several proteins are aberrantly expressed on the plasma membrane as shown by Heider et al.^{[3]} Therefore importantly, the inventor is the first to show that the treatment of malignant cells with a CRBN inhibitor, or with a low concentration of the highly polar organic solvent Dimethyl Sulfoxide (DMSO), is increasing the aberrant expression of Ras protein in the cells. This enables for the combination therapy of a CRBN inhibition and/or organic solvent with an anti Ras directed immunotherapy, for a higher, more efficient and safer killing of malignant cells.

In an eighth aspect, the invention pertains to a compound for use in medicine, wherein the compound is selected from an ABP according to the present invention, a CAR according to the present invention, an isolated nucleic acid according to the present invention, an expression construct according to the present invention, a recombinant host cell according to the present invention, a pharmaceutical composition according to the present invention, and a pharmaceutical combination according to the present invention, according to the present invention.

In a ninth aspect, the invention pertains to a compound for use in the diagnosis, prevention and/or treatment of a proliferative disorder, such as cancer, or for use in adoptive, target-cell specific immunotherapy, wherein the compound is selected from an ABP according to the present invention, a CAR according to the present invention, an isolated nucleic acid according to the present invention, an expression construct according to the present invention, a recombinant host cell according to the present invention, a pharmaceutical composition according to the present invention, and a pharmaceutical combination according to the present invention, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

In a tenth aspect, the invention pertains to combination of an ABP according to the present invention, a CAR according to the present invention, an isolated nucleic acid according to the present invention, an expression construct according to the present invention, a recombinant host cell according to the present invention, a pharmaceutical composition according to the present invention, and a pharmaceutical combination according to the present invention, and a chemical compound, wherein the chemical compound is an organic solvent or an inhibitor increasing the expression of Ras protein on the extracellular site of the plasma membrane, preferably wherein the chemical compound is a cereblon (CRBN) inhibitor, or a polar organic solvent like DMSO, for use in a method of diagnosis, prevention and/or treatment of a proliferative disorder, such as cancer.

In a preferred embodiment, the therapeutic compounds of the present invention are bispecific antibodies for use in the treatment of different types of cancer, such as cancer characterized by (a) mutated Ras gene(s).

In a preferred embodiment, the therapeutic compounds of the present invention are tri-specific or multi-specific antibodies for use in the treatment of different types of cancer, such as cancer characterized by (a) mutated Ras gene(s).

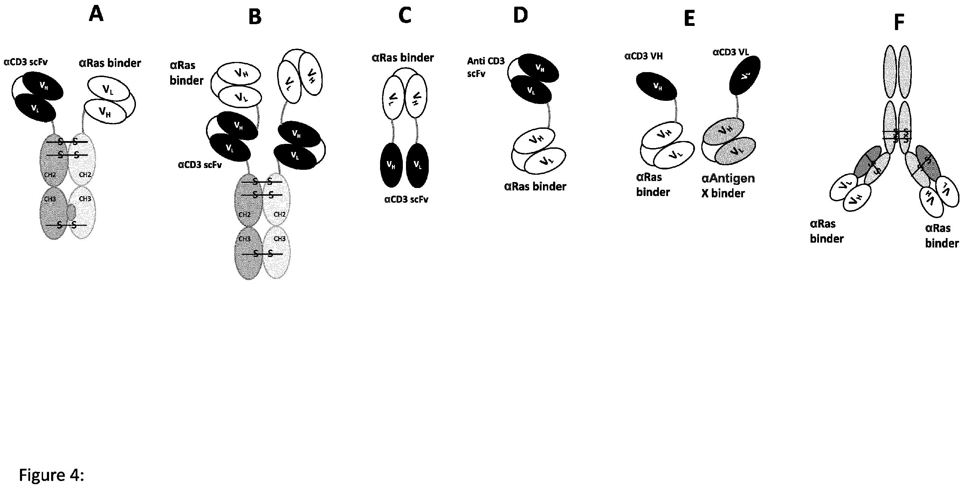

Examples of tri-specific antibodies according to the invention are shown in Figures 4 and 5.

Importantly, target-cell specific immunotherapy is a very promising treatment strategy for, e.g., the treatment of cancer. When using target-cell specific immunotherapy, endogenous cells of the patient are used to destroy cancer cells via induction of an immune response, whereas treatment strategies for cancer not based on immunotherapy often use external compounds to destroy cancer cells. It is often difficult to control targeting just cancer cells using such external compounds, and such treatment strategies, therefore, risk harming healthy cells. Therefore, target-cell specific immunotherapeutic treatment strategies are highly advantageous over the use of external compounds.

In a preferred embodiment, the cancer is metastatic, stage III cancer, or stage IV cancer, optionally wherein the cancer is a Ras-dependent cancer.

In another embodiment, which can be combined with any aspect and any other embodiment of the present invention, the compounds of the present invention are for use in the diagnosis, prevention and/or treatment of RASopathies. RASopathies are a heterogenic group of developmental conditions, in which germline mutations in the Ras gene induce an overactivation of the RAS-MAPK signaling cascade. The result of an overactivation of the RAS-MAPK signaling cascade can be a malformation of the heart or other organs, an impairment of growth or developmental defects, and an enhanced risk for cancer.

In a preferred embodiment, which can be combined with any aspect and any other embodiment of the present invention, a disorder to be diagnosed, prevented and/or treated by any of the compounds of the present invention is a RAS driven disorder. Thus, the present invention provides antidromic strategies to target various RAS driven disorders.

In one embodiment, the compound for use according to the eighth aspect of the present invention is for use in modulating a cell-mediated immune response in a subject, optionally wherein the modulating the immune response is an inhibition of a cell-mediated immune response.

A further embodiment relates to the above compound for use according to the eighth aspect of the present invention wherein the inhibition of a cell-mediated immune response is an inhibition of proliferation of an immune cell, such as a lymphocyte, and/or is an inhibition of cytokine expression in an immune cell, such as a lymphocyte.

Another embodiment pertains to the above compound for use according to the eighth aspect of the present invention wherein the inhibition of a cell mediated immune response is a reduction of proliferation/activity of effector memory T cells and/or increase of proliferation/activity of regulatory T cells (TREGs).

Another aspect relates to a diagnostic use of the ABPs of the present invention to detect cells aberrantly expressing Ras protein on the surface of cells in vitro (for example by ELISA) or in vivo (for example by PET-CT with a Ras ligand).

A further aspect of the present invention relates to a method for the diagnosis, prevention and/or treatment of a proliferative disorder, such as cancer, in a subject, comprising the administration of a therapeutically effective amount of an ABP according to the present invention, a CAR according to the present invention, an isolated nucleic acid according to the present invention, an expression construct according to the present invention, a recombinant host cell according to the present invention, or a pharmaceutical composition according to the present invention, to the subject, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

A preferred embodiment relates to a method of diagnosis, prevention and/or treatment of a proliferative disorder, such as cancer, in a subject, comprising the administration of an ABP according to the present invention, a CAR according to the present invention, an isolated nucleic acid according to the present invention, an expression construct according to the present invention, a recombinant host cell according to the present invention, or a pharmaceutical composition according to the present invention, to the subject, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

A further aspect of the present invention relates to the use of an ABP according to the present invention, a CAR according to the present invention, an isolated nucleic acid according to the present invention, an expression construct according to the present invention, a recombinant host cell according to the present invention, or a pharmaceutical composition according to the present invention for the manufacture of a medicament for the treatment of a proliferative disorder, such as cancer, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancer, skin cancer, leukaemia, lymphoma, myeloma, myeloid malignancies, lymphoid malignancies, breast cancer, ovarian cancer, neuroendocrine carcinoma, endometrial cancer, vaginal cancer, blood-related cancer, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3). One particularly preferred example of a cancer to be treated is NEC-G3 of the gastrointestinal tract or small cell lung cancer (NEC-G3 lung carcinoma).

Yet another aspect of the present invention pertains to the use of an ABP according to the present invention, a CAR according to the present invention, an isolated nucleic acid according to the present invention, an expression construct according to the present invention, a recombinant host cell according to the present invention, or a pharmaceutical composition for generating target-specific effector cells.

In one embodiment, the present invention pertains to the use of an ABP according to the present invention, a CAR according to the present invention, an isolated nucleic acid according to the present invention, an expression construct according to the present invention, a recombinant host cell according to the present invention, or a pharmaceutical composition for generating cytotoxic T cells.

A further aspect of the present invention relates to an ABP according to the present invention, a CAR according to the present invention, an isolated nucleic acid according to the present invention, an expression construct according to the present invention, a recombinant host cell according to the present invention, and a pharmaceutical composition for use in killing tumor cells.

Yet another aspect of the present invention relates to a method of modulating a cell-mediated immune response in a human cell that expresses an extracellular Ras antigen, comprising contacting said cell with a an ABP according to the present invention, a CAR according to the present invention, an isolated nucleic acid according to the present invention, an expression construct according to the present invention, a recombinant host cell according to the present invention, a pharmaceutical composition, or a compound for use according to the present invention, in the presence of an immune cell, such as a T-cell, thereby modulating, preferably enhancing, the cell-mediated immune response.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

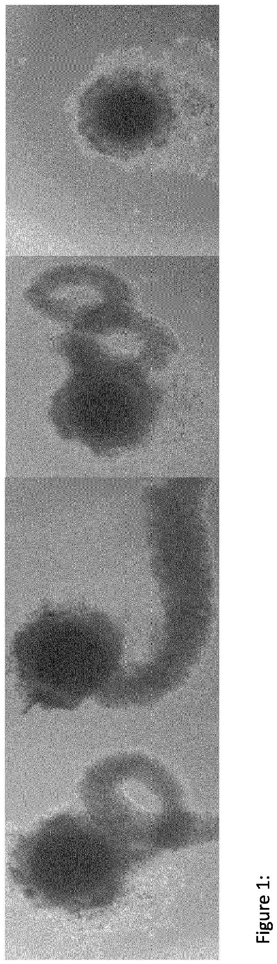

Figure 1 shows for the new primary patient neuroendocrine carcinoma (NEC-G3) cell line in cell culture suspension the agglomeration and formation of loosely adhesive cell spheroids with long worm like protuberances.

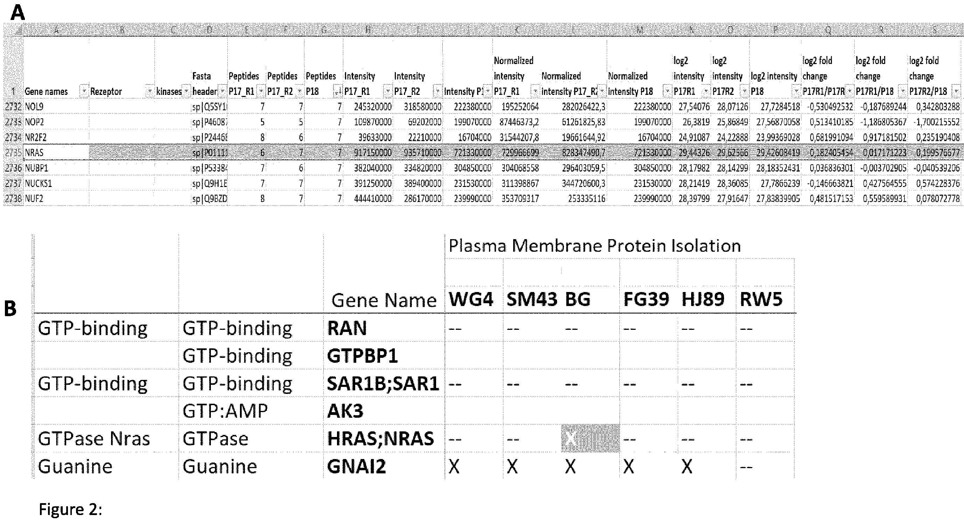

Figure 2A shows high expression of NRAS proteins in cells of a NET-G3 carcinoma using mass spectrometry analysis for protein peptide fragments in cell lysate. Figure 2B shows the expression of HRAS/NRAS proteins in the plasma membrane of a patient primary CD138+ Multiple Myeloma cells using mass spectrometry analysis for protein peptide fragments (the positive HRAS/NRAS signal is highlighted by a grey box with a white X for patient BG).

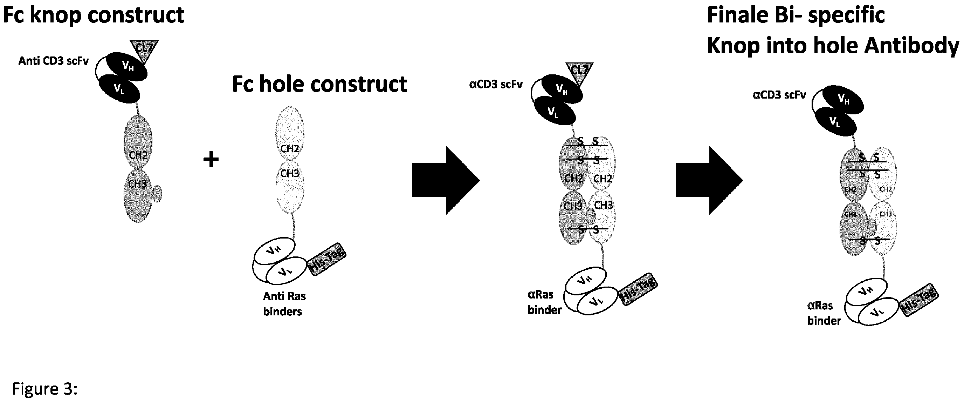

Figure 3 shows the antibody knop into hole format for a bi-specific T cell engaging anti Ras binder. On the left side are the two different Fc constructs with different antigen binding sites. The anti(α) CD3 binder (filled black ovals) is at the N-terminal end of the IgG Fc knop construct, linked via a flexible glycine serine linker to the constant CH2 domain. At the N-terminal end of the anti CD3 scFv is a CL7 tag (CL7, grey triangle) attached, via a 3c protease linker. The anti(α) Ras binder (filled white ovals) is attached at the C-terminal end of the IgG Fc hole construct, to the constant CH3 domain. At the C-terminal end of the Ras binder is a His-tag (grey rectangle) via a glycine-serine linker attached. For recombinant production, the Fc knop and Fc hole antibody plasmids are mixed together for cell transfection. The produced heterodimer bi-specific antibody is extracted und purified using the two different protein tags at each end (middle picture). This allows for an equal ratio of paired heterodimers, and no excess of one Fc-chain with anti-Ras or anti-CD3 binders in the final product. The CL7 tag is removed during the purification process using a protein 3c protease, creating the final bi-specific knop into hole antibody targeting Ras and CD3 (right side). Straight black lines are disulfide bridges (-S-S-) and curved grey or black lines are protein linkers.

Figure 4 shows alternative antibody formats for anti-Ras binders. Figure 4A shows a hetero-dimer antibody knop into hole format with the anti(α) CD3 (filled black ovals) and anti(α)Ras (filled white ovals) binders are both attached at the N-terminal end of the antibody. Figure 4B shows the anti CD3 (filled black ovals) and anti-Ras (filled white ovals) binders connected to the same antibody constant chain, creating a bi-specific homodimer. Figure 4C shows a bi-specific single chain diabody format for the anti-CD3 (filled black ovals) and anti-Ras (filled white ovals) binders. Figure 4D shows a bi-specific BiTE antibody format for the anti-CD3 (filled black ovals) and anti-Ras (filled white ovals) binders. Figure 4E shows a dual antigen restricted tri-specific hemibody format for the anti-CD3 (filled black ovals), anti-Ras (filled white ovals) and against a second tumour antigen anti AntigenX (filled grey ovals). This hemibody pair is redirecting CD3 positive T cells against a dual antigen positive target cell. The first target cell antigen is Ras and a second antigen (antigen X) can be any antigen expressed on the plasma membrane of the target cell. Figure 4F shows an IgG antibody with the variable domains against Ras (white) (the antibody constant heavy chains (CH1, CH2 and CH3) are shown in light grey and the antibody constant light chain (CL) are shown in dark grey). Straight black lines are disulfide bridges and curved grey or black lines are protein linkers.

For each of the anti(α)Ras (filled white ovals) binders in Figures 3, 4 and 5, different antigen binding formats can be used: (i) antibody scFv chains in different orientation (V_{H}-V_{L}) or (V_{L}-V_{H}), (ii) single chain VHH (single chain antibody, Nanobody) binders, (iii) armadillo repeat proteins (ArmRP), (iv) novel synthesized (computer based and/or artificial intelligence optimized) protein peptides with Ras binding, (v) protein peptides from human proteins with known Ras binding sites or (vi) designed ankyrin repeat proteins (DARPins). All Ras binders in Figures 3, 4 and 5 can be specific to bind: (i) only to single, more or all of the different native human Ras proteins (SEQ ID NOs: 1, 3, 5, 7, or 9), (ii) or only to single, more or all of the different mutated human Ras proteins (SEQ ID NOs: 11-19 and 77-119), (iii) or both, to one more native and mutated human Ras proteins, or to splice variants thereof.

Figure 5 shows alternative anti-Ras directed immunotherapy formats. Figure 5A shows on the left a single antigen targeting chimeric antigen receptor (CAR) T-cell against Ras (filled white ovals). On the right, a dual antigen targeting CAR T-cell against two different antigens is shown, *i.e.* Ras (filled white ovals) and a second antigen (filled grey ovals) (antigen X), wherein the second antigen can be any antigen expressed on the extracellular plasma membrane of the target cell to increase specificity, avidity and effectiveness against the tumours. Figure 5B shows a single chain KeyLock antibody, targeting Ras (T1, black spotted ovals) and a second target cell antigen (T2, black wave ovals), covalently connected by a linker (gray solid line). The anti-CD3 receptor complex directed effector binding domain (F) of an anti-CD3 antibody is split/separated into the VH fragment (F1 light grey) at the NH2-terminus and into the VL fragment (F2 dark grey) at the COOH terminus with a short 6 amino acid His-tag (H) for purification. In the KeyLock antibody format, the orientation (from the NH2-terminus to COOH-terminus) of the different connected antibody variable domains can be in the V_{H}-V_{L} and/or the V_{L}-V_{H} orientations, with the split/separated antiCD3 V_{H} and/or V_{L} domains connected. Figure 5C shows a ligand connected to an anti(α)Ras (black wave ovals) binder. The ligand can be any chemical or protein ligand, or both, for detection or depletion of extracellular Ras expressing cells in the patient. Figure 5D shows two different virus type immunotherapies with a Ras binding site. The virus can be any type of oncolytic viruses (DNA based or RNA based virus) for clinical use in humans.

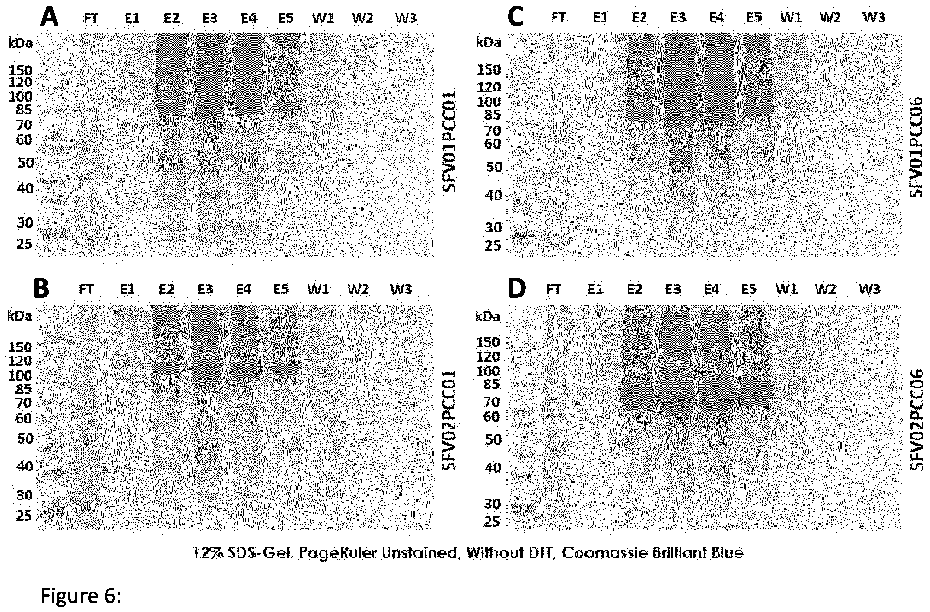

Figure 6 shows the first step in the recombinant production process of four different anti Ras antibodies, in the knop into hole format, using eukaryotic cells. Using the His-Tag of the antibodies, the CO-IMAC purified anti Ras antibodies are loaded onto a 12% SDS-PAGE Gel and stained with Coomassie Brilliant Blue (CBB). Figure 6A shows the SFV01_PCC01 antibody, Figure 6B shows the SFV02_PCC01 antibody, Figure 6C shows the SFV01_PCC06 antibody and Figure 6D shows the SFV02_PCC06 antibody. Abbreviations: Column flow through (FT), column washing fractions (W1, W2 and W3), antibody elution fractions (E1, E2, E3, E4 and E5), Dithiothreitol (DTT). A PageRuler^{™} Unstained Protein Ladder was used as marker on the left. Kilo-Dalton (kDa).

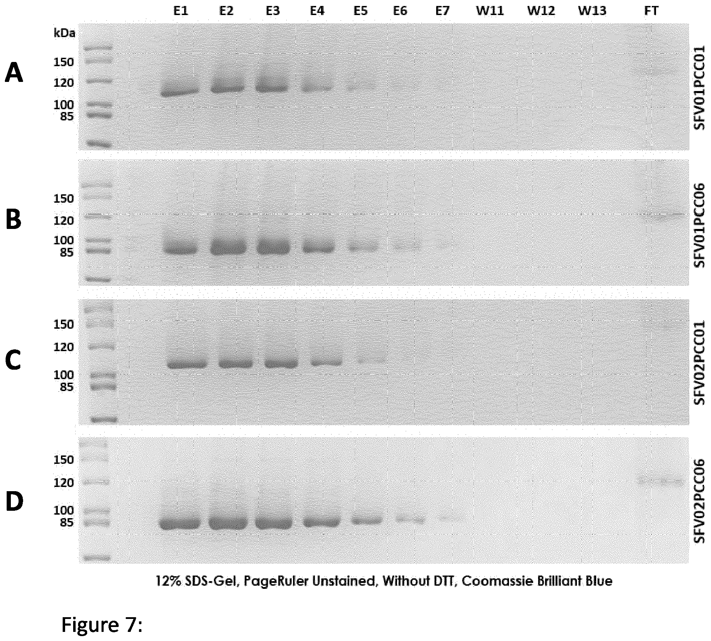

Figure 7 shows the second step in the recombinant production process of four different anti-Ras antibodies. Using in this second step the CL7 tag for purification, the final purified anti Ras antibodies are loaded onto a 12% SDS-PAGE Gel and stained with Coomassie Brilliant Blue (CBB). Figure 7A shows the SFV01_PCC01 (SEQ ID No 73 + 74) antibody, Figure 7B shows the SFV01_PCC06 (SEQ ID No 73 + 76) antibody, Figure 7C shows the SFV02_PCC01 (SEQ ID No 75 + 74) antibody and Figure 7D shows the SFV02_PCC06 (SEQ ID No 75 + 76) antibody. Abbreviations: Column flowthrough (FT), column washing fractions (W11, W12 and W13) and antibody elution fractions (E1, E2, E3, E4, E5, E6 and E7), Dithiothreitol (DTT). A PageRuler^{™} Unstained Protein Ladder was used as marker on the left. Kilo-Dalton (kDa).

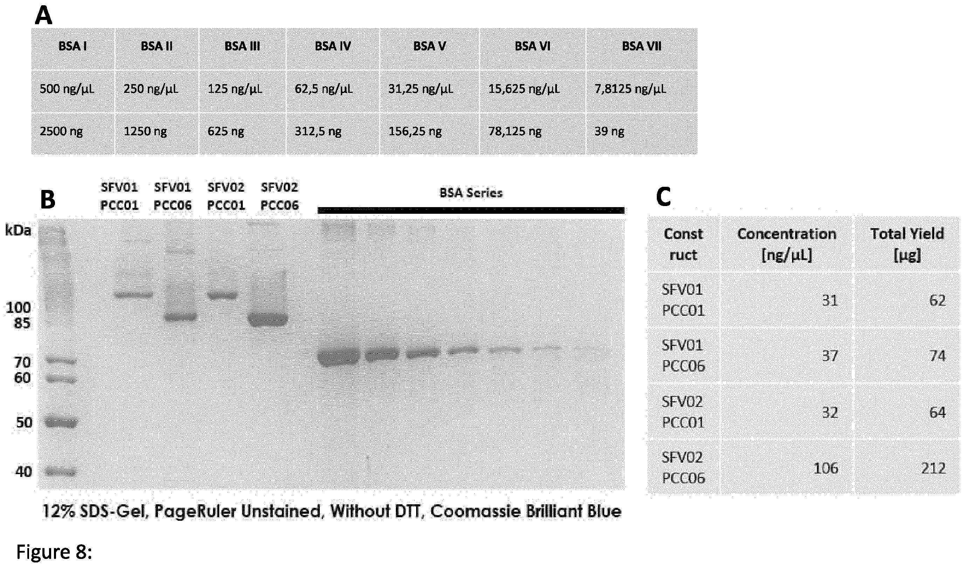

Figure 8 shows the final purified anti Ras antibodies with high purity and protein yields. Figure 8A shows a table for the protein concentrations of the bovine serum albumin (BSA) standard used to determine the recombinantly produced antibody concentrations (BSA I to VII). Figure 8B shows the four different anti Ras antibodies, and a bovine serum albumin (BSA) protein concentration control, loaded onto a 12% SDS-PAGE Gel and stained with Coomassie Brilliant Blue (CBB). Figure 8C shows the calculated protein concentration for the four different anti Ras antibodies and the total final production yield.

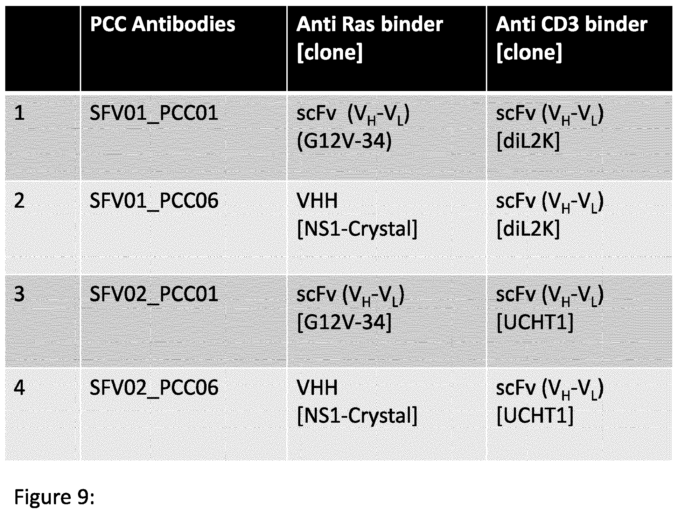

Figure 9 shows a table for the four anti-Ras antibodies and the different binding sequences used. For anti-Ras binders, two different binding sequences were used. (i) From the G12V-34 clone, the single chain variable fragment (scFv) with a variable heavy (V_{H}) domain connected via a 15 amino acid long flexible glycine serine (GS) linker to the variable light (V_{L}) domain^{[4]}. (ii) From the NS1-crystal clone (5E95, Crystal Structure of Mb(NS1)/H-Ras Complex)^{[5, 6]}, a single domain (VHH) binder was used. For anti-CD3 receptor complex epsilon chain binders, two different scFv binding sequences were used (from the diL2K clone and the UCHT1 clone)^{[7,8]}. For both anti-CD3 scFv binders, the variable heavy (VH) domain is connected via a 15 amino acid long flexible glycine serine (GS) linker to the variable light (VL) domain.

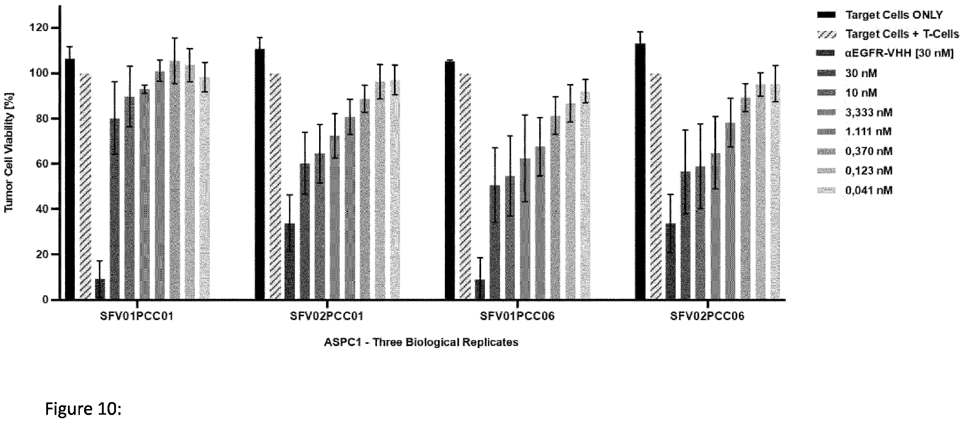

Figure 10 shows that four anti Ras bispecific antibodies - SFV01_PCC01 (SEQ ID No 73 + 74), SFV01_PCC06 (SEQ ID No 73 + 76), SFV02_PCC01 (SEQ ID No 75 + 74) and SFV02_PCC06 (SEQ ID No 75 + 76) - have a high activity against the human pancreas carcinoma cell line ASPC1, in a dose dependent manner from 30 nM to 0.37 nM. Shown are the results of three independent experiments. A bispecific antibody against EGFR (αEGFR-VHH) was used as a positive control for bi-specific antibody mediated T cell killing of target cells. Tumour cells only (Target cells ONLY) and tumour cells with CD8+ T cells (Target cells + T-cells) were used as negative control and to exclude a possible T-cell response against neoantigens of the specific cell line. The ASPC1 cell line is a pancreas metastasic / adenocarcinoma cell line with a KRAS G12D (homozygous) mutation. In here, the bispecific antibodies SFV01_PCC06 and SFV02 _PCC06 with a single domain antiRas binding VHH site, show a better killing at the respective concentrations compared to the bispecific antibodies with an antiRas scFv binding site (SFV01_PCC01 and SFV02_PCC01).

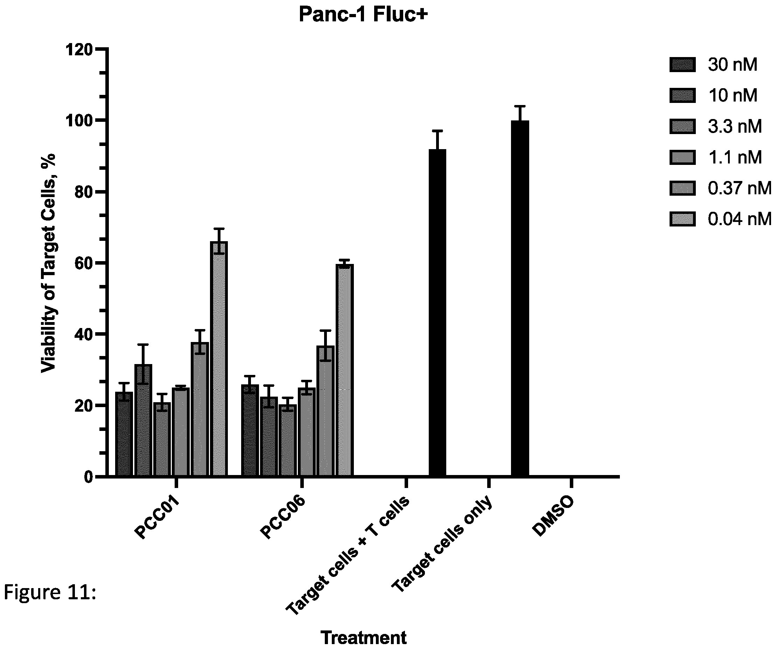

Figure 11 shows that two anti-Ras bispecific antibodies (PCC01 = SFV02_PCC01, and PCC06 = SFV02_PCC06) exhibits strong activity against the luciferase positive (Fluc+) human pancreas carcinoma cell line Panc-1, in a dose dependent manner from 30 nM to 0.37 nM. A 10% DMSO concentration in the cell culture medium was used as a positive control for tumour cell killing (DMSO). Tumour cells only (Target cells only) and tumour cells with CD8+ T cells (Target cells + T-cells) were used as negative control and to exclude a possible T-cell response against neoantigens of the specific cell line. The PANC1 cell line is a Pancreas duct / epitheliod carcinoma cell line with a KRAS G12D (heterozygous) mutation.

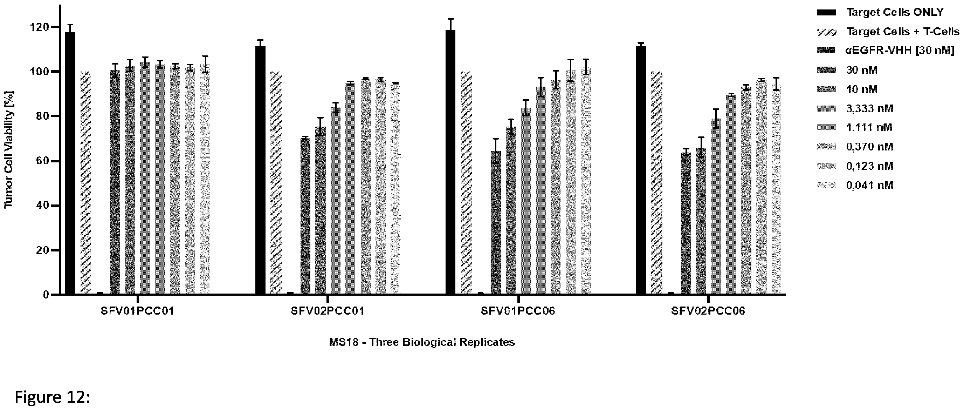

Figure 12 shows that three anti-Ras bispecific antibodies (SFV02_PCC01, SFV01_PCC06 and SFV02_PCC06) have activity against the human neuroendocrine carcinoma cell line MS18, in a dose dependent manner from 30 nM to 1.111 nM. Shown are the results of three independent experiments. A bispecific antibody against EGFR (αEGFR-VHH) was used at a final concentration of 30 nM as a positive control for bi-specific antibody mediated T cell killing of target cells. Tumour cells only (Target cells ONLY) and tumour cells with CD8+ T cells (Target cells + T-cells) were used as negative control and to exclude a possible T-cell response against neoantigens of the specific cell line. The MS18 cells are liver metastasis / neuroendocrine carcinoma cells with a KRAS G12D (heterozygous) mutation.

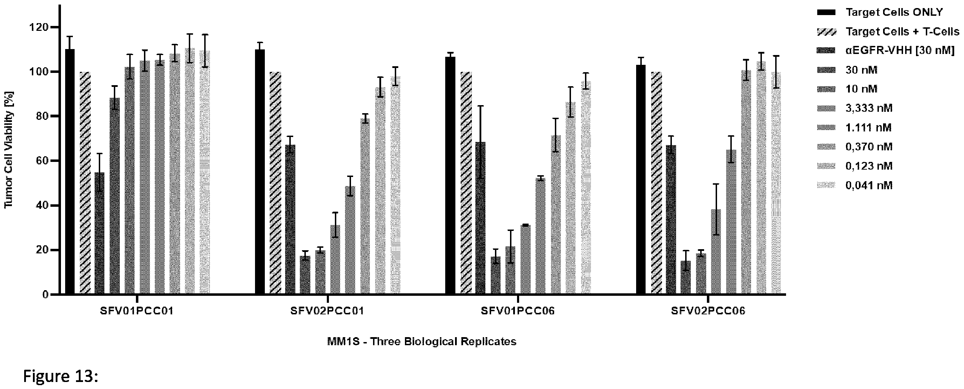

Figure 13 shows that three anti-Ras bispecific antibodies (SFV02_PCC01, SFV01_PCC06 and SFV02_PCC06) have high activity against the human Multiple Myeloma cell line MM.1S, in a dose dependent manner from 30 nM to 0.37 nM. Shown are the results of three independent experiments. A bispecific antibody against EGFR (αEGFR-VHH) was used at a final concentration of 30 nM as a positive control for bi-specific antibody mediated T cell killing of target cells. Tumour cells only (Target cells only) and tumour cells with CD8+ T cells (Target cells + T-cells) were used as negative control and to exclude a possible T-cell response against neoantigens of the specific cell line. The MM.1S cell line is a Multiple Myeloma / hematopoietic cell line with a KRAS G12A (heterozygous) mutation.

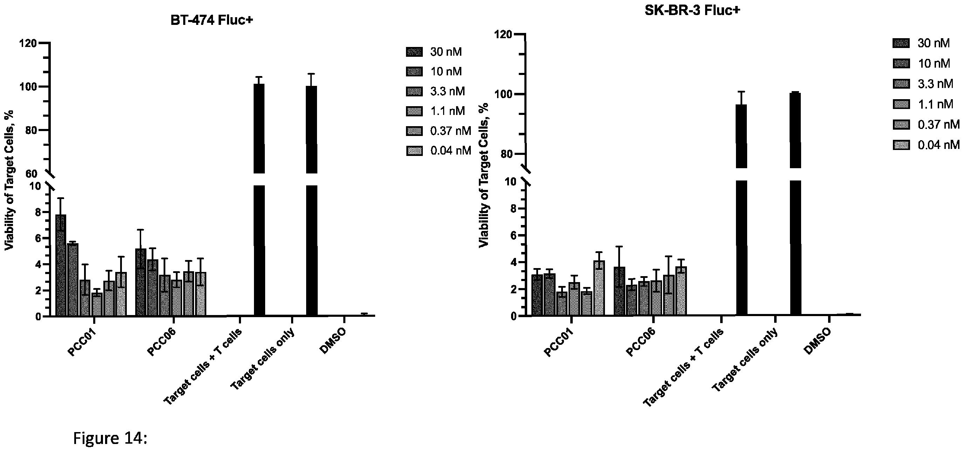

Figure 14 shows that two anti-Ras bispecific antibodies (PCC01 = SFV02_PCC01, and PCC06 = SFV02_PCC06) exhibit strong activity against the luciferase positive (Fluc+) human tumour cell lines BT-474 and SK-BR-3. BT-474 and SK-BR-3 are breast carcinoma cell lines with wild type Ras genes. But for both cell lines, the wild type Kras protein is a critical regulator for their metastatic behavior and is associated with mesenchymal features of these cancers. These data show that also cancer cells with no activating mutation in a Ras gene can express Ras proteins on their cell surface, and can be eliminated with an anti-Ras directed bi-specific antibody.

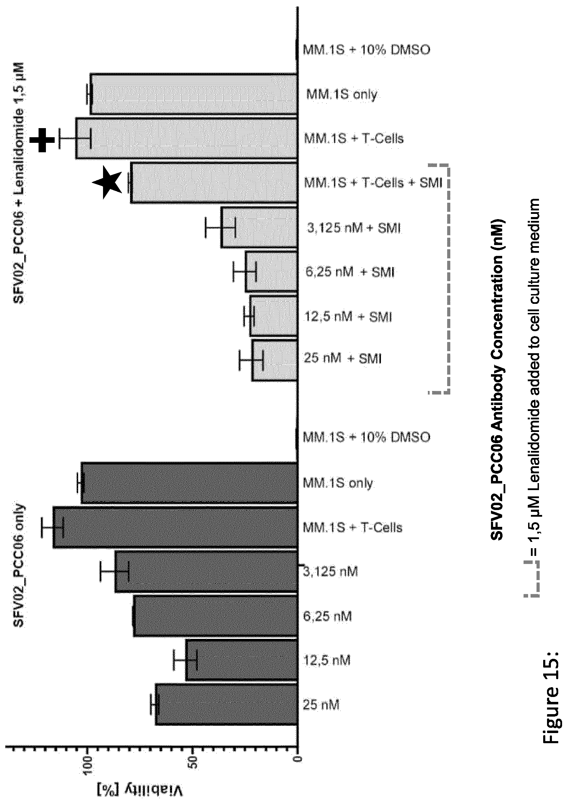

Figure 15 shows that the combination of the cereblon (CRBN) inhibitor Lenalidomide with an anti Ras bispecific antibody (SFV02_PCC06) is significantly increasing the antibody mediated killing of target cells. 10% DMSO in cell culture medium was used as a positive control for tumour cell killing (MM.1S + 10% DMSO). Tumour cells only (MM.1S only) and tumour cells with CD8+ T cells (MM.1S + T cells) were used as negative control and to exclude a possible T-cell responses against neoantigens of the specific cell line. On the left (dark grey bars), MM.1S cells + T cells are treated with different concentrations of the bi-specific anti Ras antibody SFV02_PCC06 only, the medium maximal killing rate at the highest concentration was 35% (65% viable cells). In contrast, on the right (light grey bars), the same aliquot of MM. 1S cells was incubated with the same donor T cell aliquot and the same SFV02_PCC06 production batch, but in addition Lenalidomide was added to the cell culture medium at a final concentration of 1.5 µM. Lenalidomide had at this concentration a known small negative effect on the MM.1S cell viability itself (black star) compared to the cells without Lenalidomide (black cross) (25% viability drop). To our surprise, the Lenalidomide inhibitor had an additive major effect on the anti-Ras directed antibody therapy. The medium maximal killing rate at the highest antibody concentration (25nM) was significantly increased to 80% (20% viable cells left), and at 3.125nM antibody concentration the killing was still at 65% (35% viable cells left). This combination effect was consistent significant in replicate experiments and subtracting the known inhibitory effect of the cereblon (CRBN) inhibitor Lenalidomide on MM.1S cells. The grey dotted brace shows the tumour samples treated in combination with antibody and Lenalidomide.

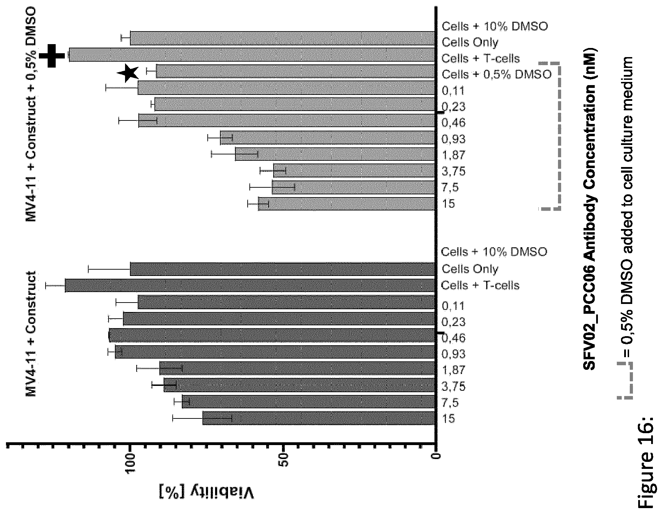

Figure 16 shows that the combination of the organic polar aprotic solvent dimethylsulfoxid (DMSO) with an anti-Ras bispecific antibody (SFV02_PCC06) is also increasing the anti-Ras directed killing of the target cells. The childhood myelomonocytic leukemia cell line MV-4-11 was used and has no mutation in the different Ras gens, but has a FMS-like tyrosine kinase-3 internal tandem duplication (FLT3-ITD), the most frequent mutations found in acute myeloid leukemia (AML). 10% DMSO in cell culture medium was used as a positive control for tumour cell killing (Cells + 10% DMSO). Tumour cells only (Cells Only) and tumour cells with CD8+ T cells (Cells + T cells) were used as negative control and to exclude a possible T-cell responses against neoantigens of the specific cell line. On the left (dark grey bars), MV4-11 cells + T cells are treated with different concentrations of the bi-specific anti Ras antibody SFV02_PCC06. The medium maximum killing rate at the highest antibody concentration (15nM) was 25% (75% viable cells left). On the right, the same aliquot of MV4-11 cells was incubated with the same donor T cell aliquot and the same SFV02 _PCC06 production batch, but in addition DMSO was added to the cell culture medium at a final concentration of 0.5% (v/v) (70.4 µM). DMSO had at this low concentration a small negative effect on the MV4-11 cell viability itself (black star) compared to the cells without Lenalidomide (black cross) (30% viability drop). To our surprise, the solvent DMSO had at this low concentration an additive major effect on the anti Ras directed antibody therapy. The medium maximal killing rate at the highest antibody concentration (15nM) was significantly increased to 45% (55% viable cells left), and at 3.75nM antibody concentration the killing was still at 50% (50% viable cells left). This combination effect was consistent significant in replicate experiments and subtracting the inhibitory effect of DMSO on cells. The grey dotted brace shows the tumour samples treated in combination with antibody and DMSO.

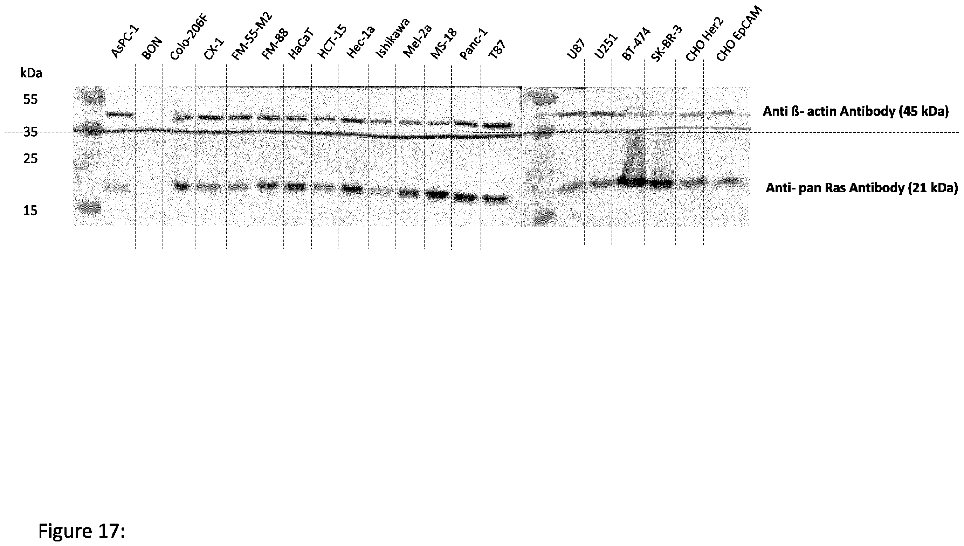

Figure 17 shows the expression of Ras proteins in different tumour cell lines total cell lysates, using Westem-blot. An antibody against human Ras protein (Pan Ras Monoclonal Antibody (Ras10)) was used. This antibody detects pan human H-, K- and N-ras proteins (at 21 kDa) and was created using the purifed recombinant Ras protein as immunogen. The antibody is also cross reactive with mouse and non-human primate Ras protein, and can be used in different applications like Western blot (WB), immunohistochemistry (IHC), immunocytochemistry (ICC/IF), flow cytometry (FACS) and immunoprecipitation (IP). An anti-β-Actin-HRP linked Antibody was used as positive control for protein loading. All tumour cell lines, with positive protein loading control, show expression of Ras proteins at the correct size in the total cell lysate. These protein lysates include cytoplasmic proteins, nuclear proteins, organelles, intra cellular plasma membrane and the extra cellular part of the plasma membrane. A PageRuler^{™} Unstained Protein Ladder was used as marker on the left. Kilo-Dalton (kDa).

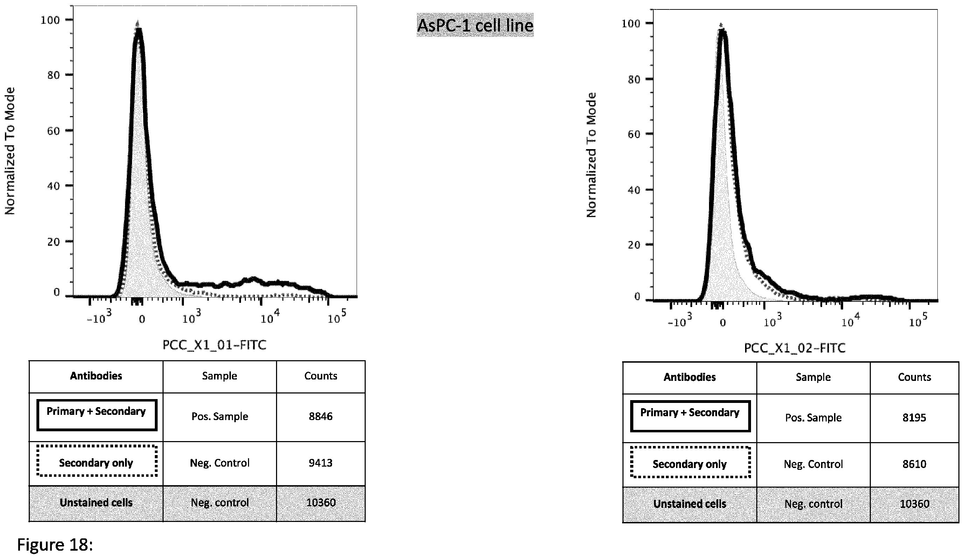

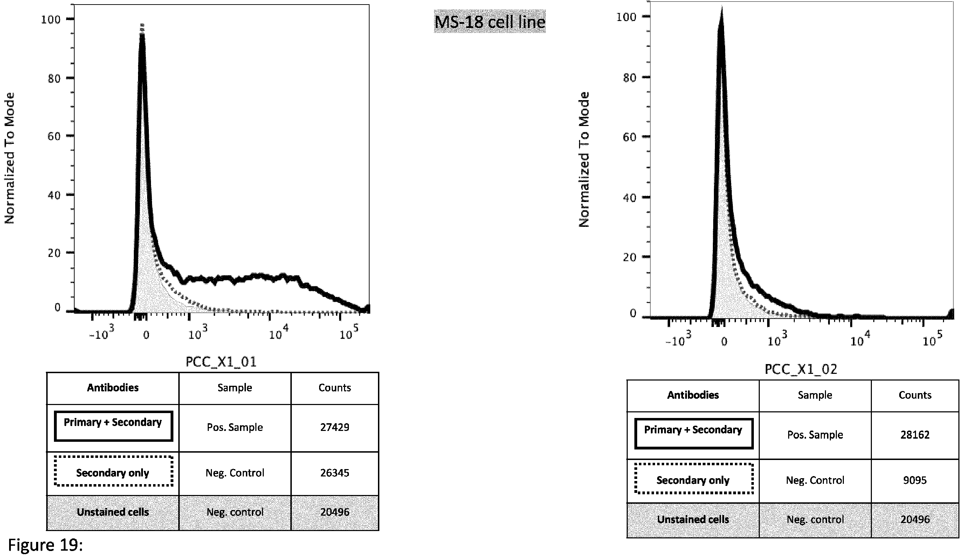

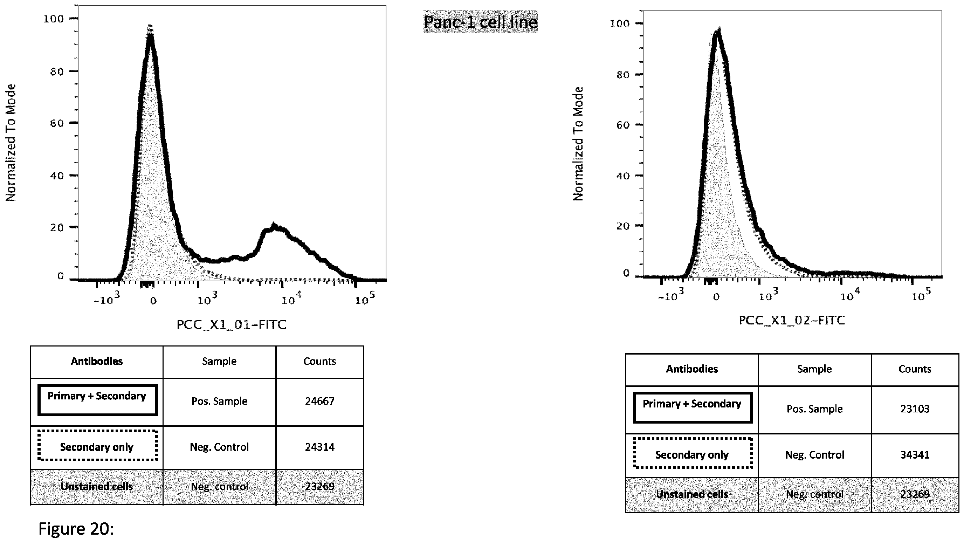

Figures 18, 19 and 20 show the aberrant expression of Ras protein on the extracellular part of the plasma membrane of different tumour cells. Three different tumour cell lines were used (Figure 18: AsPC-1, Figure 19: MS18 and Figure 20: Panc-1) to screen Ras expression on the cell surface using FACS analysis. For the FACS, only viable cells were used in the data collection, to exclude unspecific binding of anti-Ras antibody to dead or damaged cells. Two different FITC labeled antibodies against human Ras protein were used. (i) The PCC_X1_01 pan Ras monoclonal antibody (Ras10), seen on the left side of the Figures, and (ii) the PCC_X1_02 pan Ras monoclonal antibody (27H5), seen on the right side of the figures. The Ras 10 antibody, generated against purified recombinant Ras protein, has fairly equivalent recognition of all RAS isoforms and is not influenced by KRAS4B processing ^{[9]}. The 27H5 antibody is produced by immunizing animals with a synthetic peptide corresponding to residues near the amino terminus of human K-Ras. For each sample and antibody negative controls (Neg. control) to correct for (i) unspecific cell background-fluorescence and (ii) unspecific secondary antibody binding were used. In the FACS graphs (Figures 18, 19 and 20) the solid grey area shows the signal for the tumour cells unstained background-fluorescence. The black dotted line/ box shows the unspecific binding of the secondary FITC labeled anti-mouse (for PCC_X1_01) and anti-rabbit (for PCC_X1_02) antibody to the cells. A 7-AAD viability staining solution was used, to exclude dead cells from the analysis. Surprising, all three different tumour cell lines show weak to strong expression of Ras protein on the extracellular surface of the plasma membrane (black solid line/ box, Pos. sample). But only the antibody clone Ras10 showed good binding. The antibody clone 27H5 had no or only minimal binding to the cells. These data clearly show that tumour cells have aberrant expression of Ras proteins on the extra cellular cell surface, or Ras protein is accessible from the extracellular space for antibody binding. To detect this aberrant Ras expression, a specific antibody clone is needed. Not all anti Ras directed antibody clones are able to bind aberrant Ras proteins expressed on the cell surface.

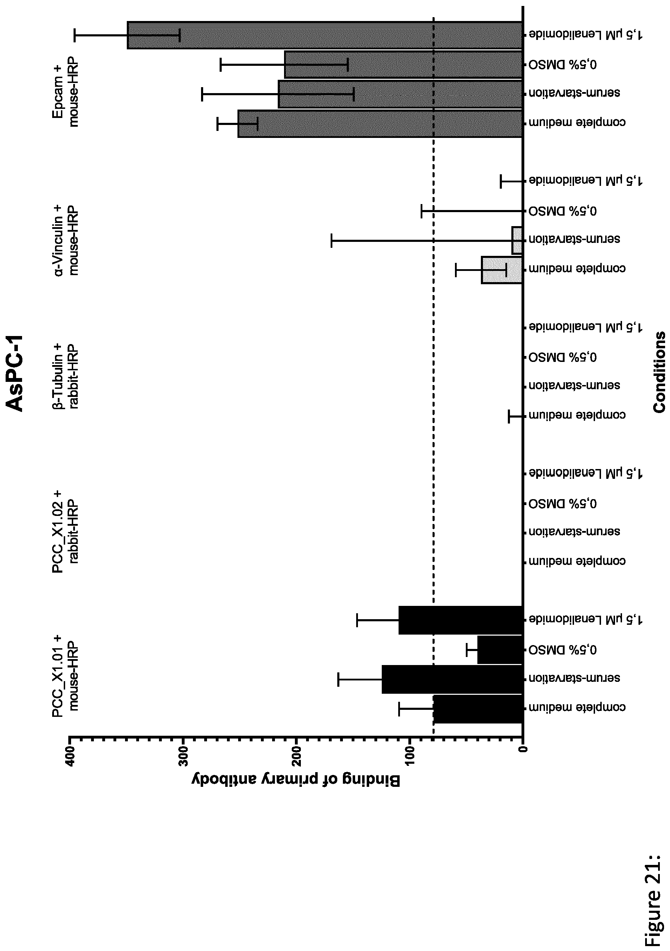

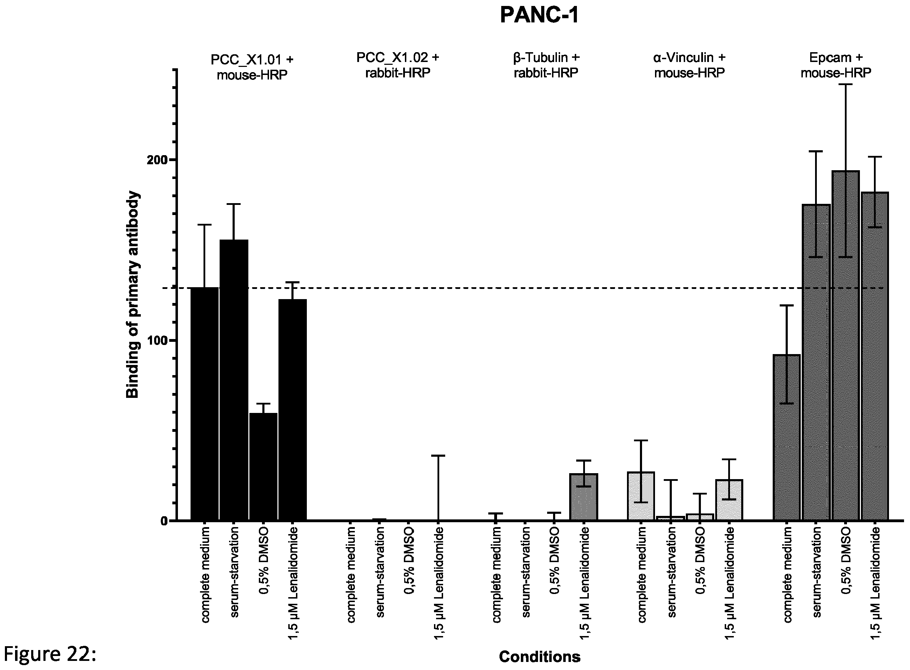

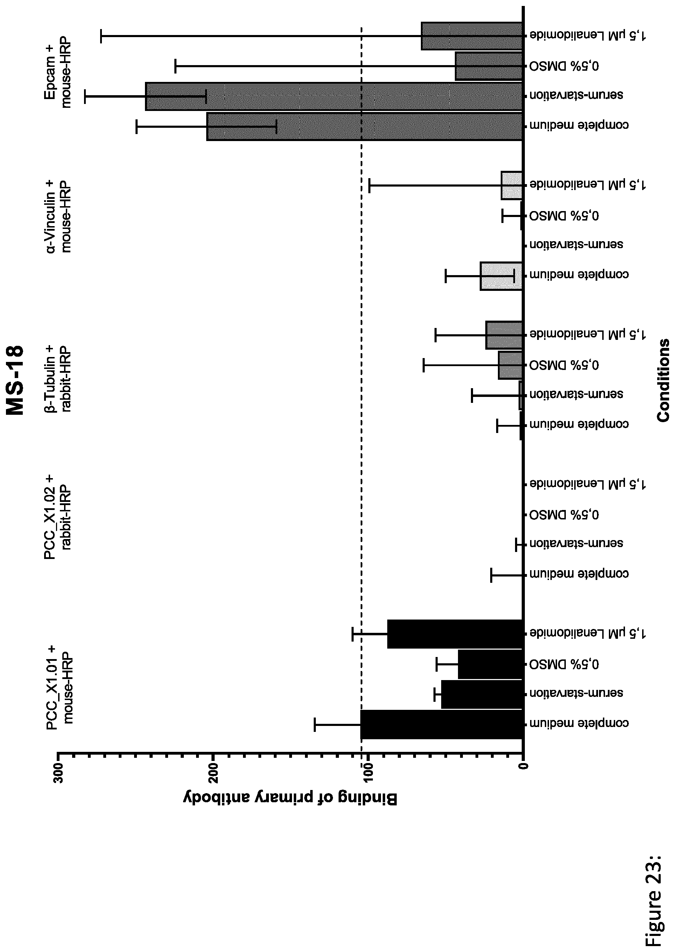

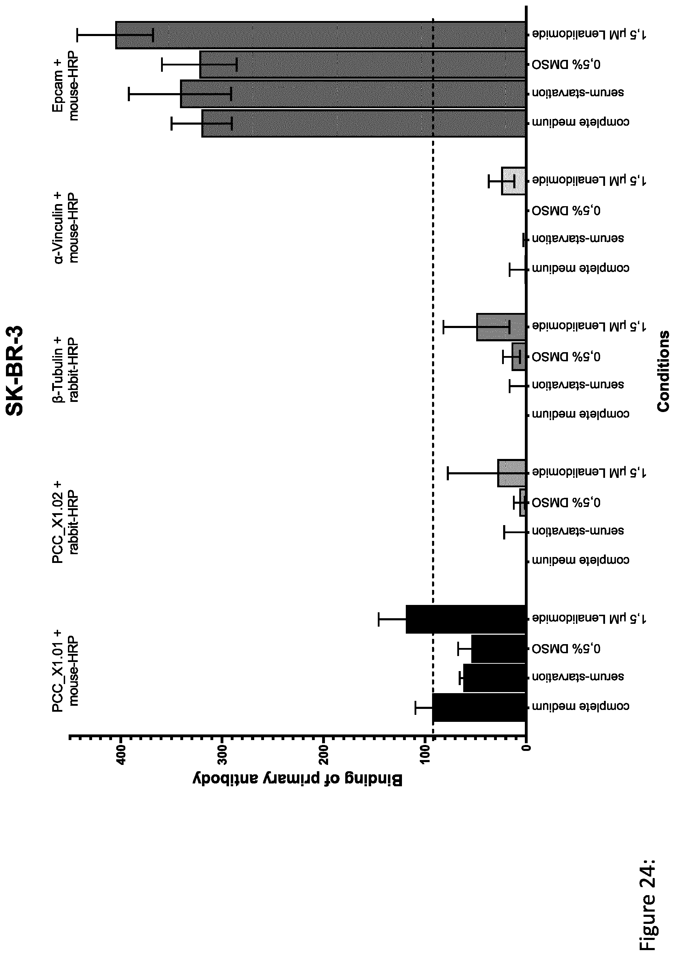

Figures 21, 22, 23 and 24 are validating the aberrant extracellular expression of Ras proteins in different tumour cell lines under different conditions, using an enzyme-linked immunosorbent assay (ELISA). Four different adherent tumour cell lines were used (Figure 21: AsPC-1, Figure 22: Panc-1, Figure 23: MS-18 and Figure 24: SK-BR-3). For detection, two different anti Ras antibodies were used (PCC_X1_01 pan Ras monoclonal antibody (Ras10), and PCC_X1_02 pan Ras monoclonal antibody (27H5)). To exclude unspecific antibody binding to intracellular proteins in damaged cells, two different antibodies against specific intracellular proteins were used, anti-Vinculin (Sigma-Aldrich, #V9131) and anti β-Tubulin (Invitrogen #MA5-16308). As a positive control for an extracellular membrane protein expressed on the cell surface of the tumour cells an anti-EpCAM antibody (Cell Signaling Technology #2929S) was used. Each cell line was cultured in four different conditions prior to the ELISA assay: (i) Cells were grown in regular cell culture medium (complete medium), (ii) cells were serum starved over-night (serum-starvation), (iii) cells were grown in regular medium with the addition of 0.5% (v/v) (70.4 µM) DMSO, (iv) cells were grown in regular medium with the addition of 1.5µM Lenalidomide. The black dotted line was set at the medium binding intensity of the PCC_X1_01 pan Ras monoclonal antibody (Ras10) to cells under normal cell culture conditions. The ELISA assay clearly validate: (i) the aberrant expression of Ras proteins on the cell surface of all four different tumour cell types under regular growing conditions. (ii) The aberrant Ras protein expression intensity various between different cell lines and cell types. The highest aberrant Ras expression on Panc-1 cells and the lowest on AsPc-1 cells. (iii) Only specific anti Ras directed antibody clones are able to bind to the aberrant expressed Ras on the cell surface. The antibody clone (Ras10). (iv) Expression of other intra cellular proteins on the tumour cell surface, proteins not directly involved in the cell membrane signaling cascade and/or scaffold proteins, is low. For alpha-Vinculin and beta-Tubulin. (v) The treatment of tumour cells with specific growing conditions, chemicals and/or inhibitors can up-regulate or down-regulate the aberrant Ras expression on the cell surface. This effect is tumour/ cell type specific. The serum starvation up-regulates aberrant Ras protein on AsPC-1 and Panc-1 cells and down-regulates it on MS-18 and SK-BR-3 cells. The organic solvent DMSO, at a low concentration, is down-regulating aberrant Ras protein on the four solid tumour cells. The cereblon (CRBN) inhibitor Lenalidomide, at a concentration of 1.5µM, is up-regulating aberrant Ras expression on AsPc-1 cells and on SK-BR-3 cells.

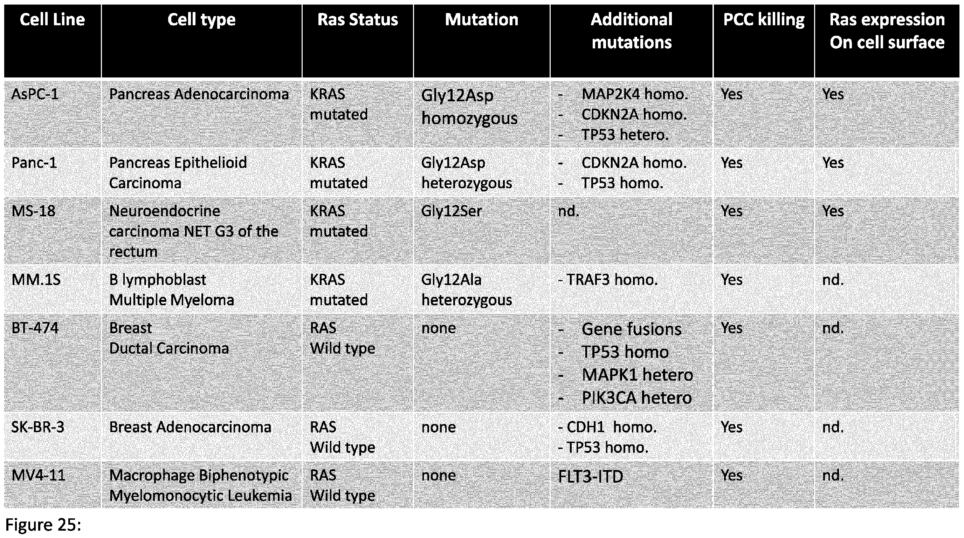

Figure 25 shows a summary table of the different cell lines used. The solid tumours (AsPC-1, Panc-1, MS-18 BT-474 and SK-BR3), the hematologic malignancies (MM.1S and MV4-11), the Ras mutated cell lines (AsPC-1, Panc-1, MS-18 and MM.1S) and the none mutated (BT-474, SK-BR-3 and MV4-11).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### 1) Establishing a novel NEC-G3 cell line

It has been a long-felt need to develop novel therapies for NEC-G3 tumors. However, so far there was no suitable human cell line available to assist in therapeutic development. The inventor was the first to derive primary tumor cells of a NEC-G3 patient, culture these cells in cell culture and to create a stable cell line out of it. The NEC-G3 patient was female, the primary tumour location site was in the rectum. The primary tumour was in the histology and immunohistology (IH) analysis high for Ki67 with 90%, the synaptophysin was high positive, chromogranin A high positive, CK 8/18 high positive, CK5/6 negative, CK7 focal weak positive, CDX2 negative. The patient had a liver metastasis with a Ki67 score of 80%, the synaptophysin high positive, a heterogenous positive expression for chromogranin, EpCAM positive, PD-L1-stain (Antibody 28-8, Abcam) negativ (TC-Score 0 after the Cologne-Score for non small cell lung cancer/ NSCLC). PD-1 also negative. CXCR4 weak positive with a reactivity of 1%. The tumour had a KRAS-G12S mutation in exon 2. From the liver metastasis a fresh sample was taken into cell culture and from these cells, a stable new NEC G3 cell line (MS18) was developed over a time of 12 months continuous growth in cell culture. Of note, MS 18 cells show an unusual growth profile in cell culture, with a characteristic formation of long worm-like spheroids (Figure 1).

### 2) Analysis of the extracellular plasma membrane proteome of different multiple myeloma patients

Importantly, for antibody-based immunotherapies, target antigens must be expressed on the cell surface. Therefore, the proteome of the human cell line MS18 and the plasma membrane proteome of primary CD138 positive cells from the bone marrow biopsies of Multiple Myeloma (MM) patients were purified with extractions kits and sent for proteomic analysis. Firstly, 2 × 10⁷ cells were harvested and washed once with ice-cold phosphate-buffered saline (PBS, 1x working solution contains 137 mM NaCl, 2.7 mM KCl, 10 mM Na₂HPO⁴, and 1.8 mM KH₂PO₄.) followed by 10 minutes of biotinylation at room temperature in 10 ml of 0.25 mg/ml EZ-Link^{™} Sulfo-NHS-SS-Biotin (Thermo ScientificTM) solution. This is an amine-reactive, cleavable biotinylation reagent, specially characterized for applications where product integrity and risk minimization are paramount. Sulfo-NHS-SS-biotin is used for cleavable attachment of biotin to extra cellular and cell surface amines, therefore only the extracellular plasma membrane proteins a labelled with a biotin-tag and can be distinct in following protein analysis from the intra cellular proteins and the rest of the intra cellular plasma membrane proteins. The cells were then pelleted through centrifugation (1300 rpm, 5 min) and washed twice with ice-cold TBS. Next, the cells were processed as recommended in the user manual of Minute^{™} Plasma Membrane Protein Isolation and Cell Fractionation Kit (Invent Biotechnologies, Inc.). The biotinylated cells were first sensitized in 500 µl Buffer A supplemented with 1 x cOmplete^{™}, Mini, EDTA-free Protease Inhibitor Cocktail (Roche) for 10 minutes on ice and then vigorously vortexed for 30 seconds. Next, the cell suspension was applied to a filter cartridge and centrifuged at 6000 × g for 30 seconds to lyse cell membrane. The cell pellet was resuspended in the collection tube and re-passed through the same filter, followed by the same centrifugation condition. The filter was then discarded and the pellet was vigorously vortexed for 10 seconds. The cell suspension was centrifuged at 700 × g for one minute to remove intact nucleic. Next, the supernatant was collected into a fresh 1.5 ml microcentrifuge tube and centrifuged at 16000 × g for 30 minutes at 4°C. The supernatant was discarded and the pellet containing the total membrane protein fraction was resuspended in 200 µl of Buffer B through thorough pipetting. The cell suspension was again centrifuged at 7800 × g for 20 minutes at 4°C. After centrifugation, the supernatant was transferred into a fresh 2 ml microcentrifuge tube containing 1.6 ml ice-cold PBS. The solutions were mixed by inverting for a few times and centrifuged at 16000 × g for 30 minutes at 4°C to pellet the plasma membrane proteins.

The inventor sent material of different growth passages of the MS-18 cell line, and patient CD138+ cells, for proteomic analysis, using an Orbitrap^{™} mass spectrometer (MS) for MS-18 cells and High-resolution Liquid Chromatography Mass Spectrometry (LC-MS) for CD138+ MM cells. This analysis showed the expression of multiple different proteins in different growth phases. Very surprisingly, MS proteomic data showed the expression of NRAS proteins in MS18 cells (Figure 2A). Importantly, the expression of HRAS/NRAS in the extracellular plasma membrane fraction was shown also for a primary patient CD138 positive myeloma cell sample (Figure 2B). Thus, the inventor concluded from the expression data that aggressive tumours might aberrantly express a small amount of Ras proteins on their cell surface, which could serve as target antigen on tumor cells. This makes Ras proteins susceptible for an anti-Ras directed immunotherapy from the extracellular side (antidrom).

### 3) Generation of firefly luciferase-positive tumor cells lines

In order to generate the human tumour reporter cell lines, a lentiviral vector, PJ01668-eGFP-ffluc-epHIV7 was used to produce eGFP-firefly luciferase-positive cells. Native tumour cells (Table 1) were first treated with 5 µg/ml Polybrene (Sigma-Aldrich), which is a cationic polymer to reduce charge repulsion between virus and host cell surface. Five hundred thousand polybrene-treated cells were then seeded into a flat-bottom 24-well plate in 2.5 ml of their respective medium (final concentration 2 × 10⁵ cells/ml). Next, lentiviral vector PJ01668-eGFP-ffluc-epHIV7 was added to the cells at a MOI of 3 and gently swirled to mix. The transduced cells were then cultivated overnight at 37°C in a humidified incubator supplemented with 5% CO₂. The next day, 1.5 ml of fresh complete medium was added to each transduction and the cells were continued to be cultured for 2 more days. After that, old medium was refreshed and the cells were visually inspected for GFP signal to check for successful transduction. When GFP signal was observed, the cells were sorted in a BD FACSAria^{™} III Cell Sorter. Highly GFP-positive populations were selected, further cultivated, expanded and finally used as the reporter cell lines for the assay *in vitro* and *in vivo.*

### 4) Isolation of CD138+ cells from Multiple Myeloma patient samples

Plasma cells from Multiple Myeloma (MM) patients that are CD 13 8-positive were isolated through positive selection with CD138 magnetic MicroBeads from Miltenyi Biotec. Bone marrow samples (1 - 10 ml blood from a bone marrow puncture) from MM patients were obtained from the clinic with patient's consent. First, the mononuclear cells were separated from the other blood fractions through Ficoll density gradient centrifugation (Ficoll-Paque^{™} Plus, Cytivia) at 3000 rpm with acceleration at 5 and deceleration at 1 for 18 minutes. Afterwards the separated mononuclear cells (MNC) were collected, washed with 40ml PBS. The MNC were resuspended in 10 ml red blood cell lysis buffer (Gibco's ACK lysing buffer) and incubated for 15 minutes at room temperature. Then, the MNC were washed with 40 ml PBS and used for magnetic labelling of CD138 positive cells according to the user manual (CD138 MicroBeads, human, Miltenyi). Briefly, the number of MNC was first determined and 2×10⁷ total cells were resuspended into 80 µl of MACS buffer (PBS at pH 7.2 supplemented with 0.5% BSA and 2 mM EDTA). Then, 20 µl of CD138 MicroBeads were added to the cell suspension, mixed well and incubated for 15 minutes at 4°C. After the 15 minutes magnetic labelling, the samples were washed with 50ml MACS buffer and centrifuged at 1300 rpm for 5 minutes. The cell pellet was resuspended in 500 µl of MACS buffer and applied to a suitable magnetic separation column according to the total cell number. The unlabeled cells were washed out with MACS buffer and finally the magnetic labelled cells were eluted and collected by flushing the column with appropriate amount of MACS buffer. After the CD138-positive cells were collected, they were cryopreserved in 50% (v/v) complete RPMI1640 supplemented with 45% (v/v) FBS and 10% (v/v) DMSO. Or used directly for protein extraction.

### 5) Isolation of human CD8+ T cells from blood healthy donors

CD8+ T cells, from healthy human donors, were used as effector cells to test the *in vitro* activity of the anti Ras antibodies. First, the mononuclear cells were separated from the peripheral blood through Ficoll density gradient centrifugation (Ficoll-Paque^{™} Plus, Cytivia) at 3000 rpm with acceleration at 5 and deceleration at 1 for 18 minutes. Afterward the separated peripheral blood mononuclear cells (PBMCs) were collected and washed with 40ml PBS. The PBMCs were then subjected to red blood cell lysis with 10 ml of Gibco's ACK lysing buffer for 15 minutes at room temperature. Then, the PBMCs were washed once with 40 ml PBS and proceed for magnetic selection according to the user manual of the magnetic beads provided by the company (CD8+ T Cell Isolation Kit, human, Miltenyi). A negative selection was used to isolate the CD8+ T cell population from the CD8 negative PBMC cells. Briefly, the number of PBMNCs was first determined and 1× 10⁸ PBMNCs were resuspended in 400 µl of MACS buffer (PBS at pH 7.2 supplemented with 0.5% BSA and 2 mM EDTA). Then, 200 µl of MicroBeads (to deplete CD8 negative cells) was added to the PBMNCs suspension, mixed well and incubated for 15 minutes and then washed with 50 ml MACS buffer and resuspended in 500 µl of MACS buffer. The labelled PBMNC were then loaded onto their respective columns according to the cell number. All magnetic labelled CD8-negative cells were captured on to the magnetic column while the CD8-positive cells were eluted by washing the column with MACS buffer. The CD8-positive cells were then collected and cultivated in RPMI1640 supplemented with 10% (v/v) FBS, 1% (v/v) GlutaMAX^{™}, 1% (v/v) penicillin-streptomycin-neomycin antibiotic mixture and 50 IU/ml human IL-2. In order to expand the cells, Gibco^{™} Dynabeads^{™} Human T-Activator CD3/CD28 for T Cell Expansion and Activation was added to the CD8+ T cells with one bead to five cells ratio and the expansion was carried out for five days. After the 5 days, the CD3/CD28 Dynabeads^{™} were captured and removed from the proliferating CD8 positive T cells, using a magnet. The CD8+ T cells were used directly for *in vitro* killing assays or cryopreserved in 50% (v/v) complete RPMI1640 supplemented with 45% (v/v) FBS and 10% (v/v) DMSO for future use.

### 6) Cloning of anti-Ras antibodies in expression plasmids

For the development of bi-specific anti-Ras antibodies, single-chain variable fragment (scFv) sequences are necessary. Three different scFv sequences (G12V-34, G13D-18 and KGH-R1) and two heavy-chain variable domains (VHH) (NS1-crystal and NS1-patent) against Ras have been found in the literature. Additionally, isolated protein domains of different human proteins with known Ras-binding sites were used (RAF1, SOS1, SOS2, RASGPR3, RASGRF1, RASGRP1 and RASGRP2). For binding, activation and redirecting human T cells, as effector cells for destruction of Ras positive target cells, two different anti-CD3 antibody sequences were used (clone UCHT1 and clone diL2k). Both of them bind to the epsilon chain of the CD3 receptor complex, and are able to activate T cells for target cell killing via this binding. For antibody stability, the constant domains (CH2 and CH3) of a human IgG antibody was used. Because the main effector function of these bi-specific anti-Ras antibodies is mediated via CD3 positive T cells, it was the goal to reduce the native antibody-dependent cellular-cytotoxicity (ADCC), antibody-dependent cellular-phagocytosis (ADCP) and complement-dependent cytotoxicity (CDC) of the constructs. Specific effector silencing point mutations in the CH2 and CH3 domains are well known in the literature and in clinically used antibodies to reduce and/or eliminate the interaction of the antibody with multiple human Fcy receptors (FcyR) and complement proteins. The CH2 and CH3 domains were mutated with: (i) Effector silent mutation L234A/L235A and P329A for both CH2-CH3 chains, (ii) Aglycosylation mutation N297A for both CH2-CH3 chains.

The final bi-specific anti Ras antibody is a heterodimer, comprising on one Fc chain at the N-terminal end the anti-CD3 binder and on the second Fc chain at the C-terminal end the anti-Ras binder (Figure 3). Because the pairing of native antibody Fc domains is random, it is possible for eukaryote cells transfected with two different Fc plasmids, to produce different antibody Fc pairs. (i) antiCD3-Fc + antiCD3-Fc, (ii) antiRas-Fc + antiRas-Fc or (iii) antiCD3-FC + antiRas-FC. Only the correct heterodimer antiCD3-FC + antiRas-FC is the final functional bi-specific antiRas and antiCD3 antibody according to the present invention. To ensure correct pairing of the Fc domains in the recombinant production process (i.e. antiCD3-FC + antiRas-FC), we used the well known Fc knop into hole technique. This technique is using specific point mutations, allowing only the pairing of a knop Fc with a hole Fc. These mutations were: (iii) Knob mutation T366W in one CH2-CH3 chain (creating the Fc knop plasmid) and the pairing (iv) Hole mutation T366S/L368A/Y407V in the second CH2-CH3 chain (creating the Fc hole plasmid). For more antibody Fc stability an additional disulfide bridge is inserted in the constant Fc domains with a (v) mutation S354C in the Fc knop plasmid and (vi) a mutation Y349C in the Fc hole plasmid.

The bi-specific antibodies were designed to carry two different tags for better purification of the final hetero-dimer antibodies. A CL7-tag covalently connected via a 3C-protease site to the N- terminal end of the Fc knop construct (with the anti CD3 scFv binder) and an 8 amino acid long histidine affinity tag, connected via a short and flexible glycine-serine linker, to the C-terminal end of the Fc hole construct (with the anti Ras binder). The tags are used to facilitate identification and purification upon expression of the recombinant proteins. The CL7 tag is removed from the final antibody in the purification process, while the histidine affinity tag is remaining on the antibody and can be used for purification.

To generate these different antibody DNA sequences, custom gene synthesis services including chemical synthesis was used (GeneArt^{®}, Thermo Fisher Scientific Inc.). Each DNA plasmid had their specific restriction enzyme cloning sites at the end for subcloning into the expression plasmids. The final sequences were cloned into an eukaryotic expression plasmid (pCEP4 Mammalian Expression Vector, Invitrogen^{™}), and the inventor thereby developed 12 different anti-Ras bispecific T-cell recruiting antibodies (PCC01 to PCC012). Standard methods of molecular biology were used (see, e.g. Sambrook et al., Molecular Cloning: A Laboratory Manual, Cold Spring Harbor Laboratory Press, New York (2001)).

### 7) Production of plasmids in bacteria

The pCEP4 Mammalian Expression Vector (Invitrogen, V04450) carrying the respective individual antibody sequence (SFV01_PCC01, SFV01_PCC06, SFV02_PCC01 and SFV02_PCC06) was transformed separately for each sequence into *E*. *coli* Mach1 cells (Invitrogen, C862003). After short thawing *E. coli* cells on ice, 10 µL of pCEP4 antibody plasmid (10-100 ng DNA) was mixed with 90µL of cells by gentle pipetting and incubated for 20 minutes on ice. Afterwards, a heat-shock was performed at 42°C for 90 seconds followed by incubation on ice for 5 minutes. The cells were then transferred into 600 µL of 2xYT medium (Carl Roth, X966.3) and incubated at 37°C and 230 rpm for one hour. After a subsequent centrifugation at 13000 rpm for two minutes, 500 µL of the supernatant was discarded. The cells were resuspended in the remaining 200 µL of medium and plated out on LB (Carl Roth, 6673.1) agar plates containing a plasmid specific selection antibiotic (100 µg/mL Carbenicillin). The plates were then incubated upside down at 37°C overnight.

On the next day, a single cell colony was picked and inoculated in 5 mL of 2xYT medium (100 µg/mL Carbenicillin) and incubated at 37°C over night. An aliquot of each over-night culture was stored at 4°C for short-term preservation. The over-night cultures were centrifuged at room temperature and 4500 g for 10 minutes after which the pCEP4 antibody plasmids were isolated according to the QIAprep Spin Miniprep Kit (QIAGEN, 27115). For the elution step, 20 µl pre-warmed UltraPure Water (Invitrogen, 11538646) was added to the middle of the column membrane and incubated at room temperature for two minutes before centrifugation at 13000 rpm for three minutes. The elution step was repeated one more time to maximize the final plasmid DNA yield. To determine the concentration and purity of the plasmids, the products were analyzed photometrically at 260nm and 280 nm using the NanoDrop^{®} 2000 (Thermo Scientific, ND-2000). The plasmids were stored at -20°C until further use.

To ensure the correct pCEP4 antibody plasmids were picked and produced, and no cross contamination happened in the production process, 20 µl (300 - 800 ng DNA) of each plasmid was send for DNA sequencing by LGC Genomics (Berlin, Germany), using the sequencing primers pCEP4_Fwd (5'-GCGTGTACGGTGGGAGGTCTATATA-3'), SV40_Rev (5'-CACTGCATTCTAGTTGTGGTT-3') and pGEX3_Rev (5'-CCGGGAGCTGCATGTGTCAGAGG-3').

For large-scale plasmid amplifications, short-term preserved *E. coli* Mach1 cultures, positive for the respective pCEP4 anti Ras antibody plasmid, were inoculated in 500 mL of 2xYT medium (100 µg/mL Carbenicillin) and incubate at 37°C and 230 rpm overnight. An aliquot (1 ml) of each overnight culture was mixed with the same volume of LB-medium (+ 30% glycerol) and stored at -80°C for long-term preservation. The overnight cultures (499 ml) were centrifuged at 5000 g and 4°C for 30 minutes after which the plasmids were isolated according to the NucleoBond Xtra Maxi Kit (Maschery Nagel, 740414.50) while considering low copy numbers. The final purified pCEP4 antibody plasmids were reconstituted in UltraPure Water (Invitrogen, 11538646). To determine the concentration and purity of the plasmids, the products were analyzed photometrically at 260nm and 280 nm using the NanoDrop^{®} 2000 (Thermo Scientific, ND-2000). The plasmids were stored at -20°C until further use.

### 8) Recombinant production of anti-Ras bispecific antibodies

Eukaryotic cells were transfected with pCEP4 Mammalian Expression Vector (Invitrogen^{™}) carrying the respective antibody sequence. For the transient production of antibodies, Expi293F^{™} Cells (Gibco^{™}, A14527) were used. The cells were seeded on the day of transfection in fresh FreeStyle^{™} 293 Expression Medium (Gibco, 12338018) at a concentration of 2^{∗}10⁶ cells/mL in an orbital shaker flask. The transfection composite was prepared by mixing one part of plasmids, always one pCEP4 knop plasmid (with the anti CD3 binding domain) with one pCEP4 hole plasmid (with the anti Ras binding domain) (Figure 3), with three parts of PEI in fresh FreeStyle^{™} 293 Expression Medium.

The transfection composite was incubated at room temperature for 15 minutes to allow complex formation and was then added to the cells. The cell cultures were incubated at 37°C, 5% CO2 and constant shaking at 110 rpm for five days or until cell viability dropped below 60%. After the first day of transfection, Tryptone N1 (PanReac AppliChem, A1553.1000) was added to a final concentration of 0,5% (v/v) to each cell culture. Five days after transfection, the cell cultures were centrifuged at 4200 rpm and 4°C for 30 minutes. The supernatant was transferred into dialysis tubes and dialyzed against 1 × PBS (pH 7.4) at 4 °C overnight. The supernatant (containing the bi-specific anti Ras antibody) was then transferred into falcon tubes and incubated with Co-IMAC beads (200 µL of pure beads per 50 mL of supernatant, Agarose Bead Technology, 6BCL-QHNi-X) at 4°C overnight under constant rotation. The bead-protein suspension was then centrifuged at 800 g and 4°C for 30 minutes and transferred onto a 10 mL flow column (Themo Scientific, 29924). The column flowthrough was collected (FT). Afterwards, the column with the loaded beads were washed three times with 10 mL of cooled Co-IMAC loading buffer each. The washing fractions were collected (W1, W2 and W3). For the elution, 500 µL of cooled Cobalt-IMAC elution buffer was loaded while the column was closed with a stopper. After an incubation of 5 minutes, the elution fraction with the antibodies was collected (E1). The elution step was repeated four more times (E2 - E5).

To check for correct recombinant antibody production, each sample fraction from the production process was visualized on a sodium dodecyl sulfate (SDS)-PAGE gel. Therefore, 21 µL of each fraction was mixed with 7 µL of 4x protein loading buffer (non-reducing conditions) and incubated at 95°C for 5 minutes. Afterwards, 20 µL of each sample mix was loaded onto a 12% polyacrylamide gel while the PageRuler^{™} Unstained Protein Ladder was used as marker. The electrophoretic separation was performed at 90 volt for the first 20 minutes and then at 130 volt for the final 90 minutes. Afterwards, the gels were covered with Coomassie Brilliant Blue solution for 10 minutes. To remove excess staining, the gels were transferred in de-staining solution and heated for a short time. After incubation for 10 minutes, the gels were kept in demineralized water until further use.

### 9) Purification of anti-Ras bispecific antibodies

The CO-IMAC extracted Ras antibodies were purified in a second step using the CL7/IM7-System (Trialtus Bioscience). The Ras antibody elution fractions were pooled (E1- E5) and 200 µL of pure IM7-Beads were added. The mix was incubated at 4°C overnight in rotation. The next day, the mixture was centrifuged at 800 g and 4°C for 20 minutes. The pellet was transferred onto a 10 mL column and collected. The flowthrough (FT) was saved. The column was washed with 10 mL of washing Buffer A1. Afterwards, the column was washed with 10 mL of washing buffer A1 (+ 300 mM imidazole), then with 10 mL of washing buffer A2 for three more times. For the elution of the Ras antibodies, 800 µL of elution buffer A3 with 1,25 µg/mL PreScission Protease (Trialtus Bioscience, 30-2030) was added on the column, the column was closed with a stopper, and rotated at 4°C for three hours. Afterwards, the column was eluted using gravity and the elution fraction with the Ras antibody was saved (E1). Then, another 800 µL of Elution Buffer A3 without PreScission Protease was added to the column, incubated for three minutes, and the elution fractions were saved (E2-E7) for six more times. From the different washing and elution fractions, 21 µL was mixed with 7 µL of 4x protein loading buffer and incubated at 95°C for 5 minutes. Afterwards, the protein samples were loaded onto a 12% SDS-PAGE gel and run first at 90 volt for 20 minutes and then at 130 volt for the final 90 minutes. After the run, the gels were stained with Coomassie Blue solution for 10 minutes and afterwards washed in de-staining solution for 10 minutes and then transferred into VE-water for imaging (Figure 7).

For each CL7-tag purified Ras antibody, the elution fractions (E1-E7) were pooled, and dialyzed against 1× DPBS (pH 7,4) at 4°C overnight. The next day, the dialysed samples were concentrated using Aquacide II ((Sigma Aldrich, 17851-M) tubes. D-Trehalose was added to the antibodies at a final concentration of 60 mM as a solvent and preservative. The antibody solution was sterilized through 0,2 µm filtration. The antibodies (in DPBS pH 7,4 + 60 mM D-Trehalose) were kept at 4°C until further use.

### 10) Analzying antibody purity

To determine the final bi-specific anti Ras antibody concentrations and the final antibody purity, 30 µL of each antibody (in DPBS pH 7,4 + 60 mM D-Trehalose) was mixed with 10 µL of 4× protein loading buffer and incubated at 95°C for 5 minutes. A bovine serum albumin (BSA) standard was used to determine the recombinantly produced antibody concentrations. For this, 5 µL of a BSA standard (dilution I to VII) was mixed with 4x protein loading buffer and incubated at 95°C for 5 minutes, and also loaded onto the 12% SDS-Page gel. After the antibody samples and the BSA standard was loaded onto the 12% SDS-PAGE gel, the gel was run at 90 volt for the first 20 minutes and at 130 volt for the final 90 minutes. After the run, the gels were stained with Coomassie Blue solution for 10 minutes and afterward washed in de-staining solution for 10 minutes and then transferred into VE-water for imaging (Figure 8).

### 11) Testing the in vitro activity of anti-Ras bispecific antibodies against human tumour cells

Four different anti-Ras bispecific antibody constructs (SFV01_PCC01 (SEQ ID No 73 + 74), SFV01_PCC06 (SEQ ID No 73 + 76), SFV02_PCC01 (SEQ ID No 75 + 74) and SFV02_PCC06 (SEQ ID No 75 + 76)) (Figure 9) were tested in cell culture against different human tumor cell lines (PANC1, ASPC1, MM. 1S, MS18, MV4-11, BT-474 and SK-BR3). The tumor cells were positive for the luziferase fire fly protein, expressed by viable cells only. The tumor cells were seeded into 96-well cell culture plates (7500 cells in 50 µL per well for 150 cells per µL) in their respective media (Table 1) and incubated at 37°C and 5% CO2 for 24 hours. The next day, fresh human CD3+/CD8+ T-cells were added to each well, for a final tumor cell to T cells ratio of 1 to 3. A dilution series of the anti Ras antibodies was prepared for a final concentration of 30 nM, 10 nM, 3.3333 nM, 1.1111 nM, 0.3704 nM, 0.1235 nM and 0.0412 nM. From the dilution series, 20 µL was added to each respective well.

As positive control for target cell killing, a bi-specific antibody against EGFR (αEGFR-VHH) was used at a final concentration of 30 nM (Figure 10, 11, 12 and 13), or a DMSO solution with a final concentration of 5% and/or 10% in the cell culture medium was used (Figure 14, 15 and 16). As reference control, to control for unspecific donor T cell activation against the target cells, target cells only and target cells mixed with T cells was used. The plates were incubated at 37°C and 5% CO2 overnight. The next day, 20 µL of 3.5 mM luciferin solution was added to each well (final concentration 0.5 mM) and the plates were incubate at 37°C for 30 minutes. Afterwards, the bioluminescence of the firefly-luciferase was measured, using a Tecan Spark plate reader (with an absorbance scan from 350 nm to 700 nm with an integration time interval of 1000 ms) (Figure 10, 11, 12, 13, 14, 15 and 16).

**Table 1**

| Cell Line | ATCC Number | Cell type | Medium |
|---|---|---|---|
| AsPC-1 | CRL-1682 | Pancreas Adenocarcinoma | RPMI1640 supplemented with 10% (v/v) FBS, 1% (v/v) GlutaMAX^{™}, 1% (v/v) penicillin-streptomycin-neomycin antibiotic mixture |
| MV4-11 | CRL-9591 | Macrophage Biphenotypic B Myelomonocytic Leukemia | RPMI1640 supplemented with 10% (v/v) FBS, 1% (v/v) GlutaMAX^{™}, 1% (v/v) penicillin-streptomycin-neomycin antibiotic mixture |
| MM.1S | CRL-2974 | B lymphoblast Multiple Myeloma | RPMI1640 supplemented with 10% (v/v) FBS, 1% (v/v) GlutaMAX^{™}, 1% (v/v) penicillin-streptomycin-neomycin antibiotic mixture |
| MS-18 | - | Neuroendocrine carcinoma NET G3 of the rectum | DMEM/F12 supplemented with 10% (v/v) FBS, 1% (v/v) GlutaMAX^{™}, 1% (v/v) penicillin-streptomycin-neomycin antibiotic mixture, 1% (v/v) Gibco^{™} insulin-transferrin-selenium (ITS-G) |
| Panc-1 | CRL-1469 | Pancreas Epithelioid Carcinoma | DMEM (4.5 g/L) supplemented with 10% (v/v) FBS, 1% (v/v) GlutaMAX^{™}, 1% (v/v) penicillin-streptomycin-neomycin antibiotic mixture |
| BT-474 | HTB-20 | Breast Ductal Carcinoma | RPMI1640 supplemented with 10% (v/v) FBS, 10 µg/ml human insulin, 1% (v/v) GlutaMAX^{™}, 1% (v/v) penicillin-streptomycin-neomycin antibiotic mixture |
| SK-BR3 | HTB-30 | Breast Adenocarcinoma | 80% McCoy's 5a with 10% (v/v) FBS, 1% (v/v) GlutaMAXTM, 1% (v/v) penicillin-streptomycin-neomycin antibiotic mixture |

### 12) Testing the in vitro activity of anti-Ras bispecific antibodies in combination with a small molecule inhibitor and/or DMSO against tumor cells

The anti-Ras bispecific antibody constructs SFV02_PCC06 (SEQ ID No 75 + 76) was tested in cell culture against the human Multiple Myeloma cell lines MM.1S and the human acute monocytic leukemia cell line MV4-11. The MM. 1S and MV4-11 cells are luziferase positive and were seeded into 96-well cell culture plates (for each cell line 7500 cells in 50 µL per well in 150 µL medium) in the corresponding medium (Table 1) and incubated at 37°C and 5% CO2 for 24 hours. The next day, fresh human CD3+/CD8+ T-cells were added to each well, for a final tumour cell to T cells ratio of 1 to 5. Two dilution series were prepared of the SFV02_PCC06 antibody: (i) A final concentration of 25 nM, 12.5 nM, 6.25 nM and 3.125 nM (Figure 15), and (ii) a final concentration of 15 nM, 7.5 nM, 3.75 nM, 1.87 nM, 0.93 nM, 0.46 nM, 0.23 nM and 0.11 nM (Figure 16). From the antibody dilution series, 20 µL was added to each respective well.

To test if chemical compounds can improve and/or increase the bi-specific anti Ras antibody directed killing of tumours cells, two compounds were tested. (i) The immunomodulatory and antineoplastic agent Lenalidomide (PubChem CID 216326), and (ii) the highly polar organic solvent Dimethyl Sulfoxide (DMSO, PubChem CID 679). The inhibitor Lenalidomide was used at a final concentration of 1.5 µM (Figure 15), and the solvent DMSO at a final concentration of 0.5% (v/v) (70.4 µM) (Figure 16). As positive control for tumour cell killing, high DMSO concentration (10% v/v) was used. The plates were incubated at 37°C and 5% CO2 overnight. The next day, 20 µL of the luciferin solution was added to each well and the plates were incubated at 37°C for 30 minutes. Afterwards, the bioluminescence of the firefly-luciferase was measured, using a Tecan Spark plate reader (with an absorbance scan from 350 nm to 700 nm with an integration time interval of 1000 ms).

Thus, the inventor found that the combination of the CRBN inhibitor Lenalidomide with the anti-Ras bispecific antibody SFV02_PCC06 is increasing the antibody mediated killing of a human cancer cell line (Figure 15). The inventor speculates that this effect is based on the inhibition of the CRBN/Crbl protein (Uniprot Q96SW2), which is a known target of Lenalidomide. Since CRBN/Crbl is important for the correct expression and folding of membrane proteins, inhibition of CRBN/Crbl could lead to an increase of Ras protein aberrantly expressed on the cell surface. Moreover, the inventor found that the combination of low dose DMSO with the anti-Ras bispecific antibody SFV02_PCC06 is increasing the antibody mediated killing of a human cancer cell line (Figure 16). Of note, DMSO is known to have an influence on the cell membrane due to its lipophilic and hydrophilic character, which could explain the finding that a combination of DMSO with an anti-Ras bispecific antibody is increasing the antibody mediated killing of a cancer cell line.

### 13) Testing the Ras protein expression in different tumour cell lines

A total number of 1 × 10⁶ viable cells were used for Western blot analysis of Ras expression on the protein level for different adherant cell lines: AsPC-1 (pancreas carcinoma, ATCC CRL-1682), BON (BON-1, serotonin-producing neuroendocrine tumour of pancreas, CVCL_3985), Colo-206F (colon adenocarcinoma, CVCL_1988), CX-1 (colon adenocarcinoma, CVCL_2011), FM-55-M2 (cutaneous melanoma, CVCL_C590), FM-88 (cutaneous melanoma, CVCL_9679), HaCaT (spontaneously immortalized cell line, CVCL_0038), HCT-15 (colon adenocarcinoma, CVCL_0292), Hec-1a (endometrial adenocarcinoma, CVCL_0293), Ishikawa (endometrial adenocarcinoma, CVCL_2529), Mel-2a (melanoma, CVCL_A759), MS-18 (NEC-G3), Panc-1 (pancreas carcinoma, CVCL_0480), T87 (mus musculus hybridoma, CVCL_N339), U87 (cancer cell line, CVCL_0022), U251 (cancer cell line, CVCL_0021), BT-474 (invasive breast carcinoma, CVCL_0179), SK-BR-3 (breast adenocarcinoma, CVCL_0033), CHO Her2 and CHO EpCAM (chinese hamster ovary cells expressing human Her2 or human EpCAM antigen).

Firstly, cell culture medium was removed and the cells were washed one time with PBS. Then 1 ml of 0.5% Trypsin-EDTA (Gibco, #25300054) was added to trypsinise the cells for 3 min at 37°C. When the cells had been totally trypsinised, 9 ml of complete medium (Table 1) was added to stop the trypsinisation process. Cell number of each cell line was determined via Trypan Blue staining and the appropriate number of cells were harvested and washed one time with PBS. The cells were lysed with 50 µl complete RIPA lysis buffer and vortexed vigorously for 30 seconds following 30 minutes incubation at 4°C. Next, the cell lysates were centrifuged at maximum speed for 30 minutes to separate protein of interests and the cell debris. The supernatant was collected and subjected to Western blot analysis.

| | |
|---|---|
| 1x RIPA lysis buffer: | 1 mM EDTA (CAS Nr. 60-00-4) |
| | 0.5% (w/v) Sodium deoxycholate |
| | 150 mM NaCl |
| | 10 mM NaF |
| | 50 mM Tris-HCl, pH 7.4 |
| | 1% (v/v) Triton^{®} X-100 (CAS Nr. 9002-93-1) |

One tablet of cOmplete^{™}, EDTA-free protease inhibitor-cocktail (COEDTAF-RO Roche, Merck SKU 11873580001), was dissolved in 2 ml of ultra-pure water to make a 25x stock solution and stored at -20°C until needed. Complete 1×RIPA lysis buffer was prepared freshly before use by adding 400 µl of 25x protease inhibitor cocktail solution to 9.6 ml of RIPA lysis buffer.

A Western blot protocol modified from Abcam Western blot protocol (Abeam plc., 2017) was used. A 12% SDS-PAGE electrophoresis was first performed to separate proteins in the cell lysates according to their molecular weight. In order to denature the proteins, 5 µl of 4x Laemmli buffer was added to 15 µl cell lysate and boiled for 5 minutes at 95°C. The samples then were loaded into the sample wells onto the SDS-gel. Together with 5 µl of Precision Plus Protein^{™} Dual Color Standards (Bio-rad Laboratories #1610374) at the side in the marker well. The gel was original casted in an appropriate re-usable cartridge with a stacking-gel on top of a separation-gel, and hardened over night at 5°C and covered to keep them moist. Afterward the protein samples and the protein marker were loaded into the individual chambers of the stacking gel. The gel-cartridges were submerged in the Gel-box in 1 × running buffer (1x Running buffer for 1000ml, 1.51 Gram Tris-base, 7.5 Gram Glycine, 0.5 Gram sodium dodecyl sulfate (SDS) and ddH2O to 1000ml). Stacking gel (4ml): 4% Gel percentage, ddH2O 1.4ml, 30% Acrylamide 0.5ml, 2x Stacking buffer 2.0ml (for 100ml, Tris HCL 3.035 g, SDS 0.2 g, ddH2O up to 100ml, pH 6.8), 10% APS () 0.04ml and TEMED (--) 0.004ml.

Separation gel (10ml): 10% Gel percentage, ddH2O 1.5ml, 30% Acrylamide 3.3ml, 2x Separation buffer 5.0ml (for 100 ml, Tris HCL 9.08g, SDS 0.2 g, ddHSO up to 100ml, pH 8.8), 10%APS 0.1ml and TEMED 0.01ml.

4x Laemmli buffer: 150mM Tris HCL (pH 7.0), 25% Glycerol, 12% SDS, 0.05% Bromphenol Blue (CAS Nr. 115-39-9), 6% β-mercaptoethanol (CAS Nr. 60-24-2).

After loading the gels, the first run was for 15 minutes at 90 volt than the voltage was further increased to 150 V and the gel was run for another 75 minutes. Electrophoresis was stopped when the sample loading-dye front reached the bottom of the SDS-Gel chamber.

After the gel-run, the gel was carefully extracted from the re-usable cartridge, and equilibrated in Western-blot transfer buffer until the next step (12 - 24 hours). A wet gel was carefully placed on top of a wet and activated 0.2 µm nitrocellulose membrane (Cytiva #10600001), with no air bubbles between the two membranes. The Ge-Membrane sandwich was protected from both sides with blot absorbance filter papers (100% cotton fiber), soaked in the transfer buffer, and placed into an appropriate re-usable Western-Transfer-cartridge. After the wet assembling of the Westem-cartidge with no/ or minimal air bubbles in-between, it was immersed in ice-cold transfer buffer and the protein was then blotted at 100 Volt for approximately 90 minutes. When the transfer was over, the membrane was first blocked with 5% non-fat skimmed milk (Carl Roth^{®}, CAS No. 68514-61-4) in 1×TBST for 1 hour at room temperature. Then, the membrane was incubated with primary antibody (PCC_X1_01 (Pan Ras Monoclonal Antibody (Ras10), Invitrogen #MA1-012) diluted 1:3000 in 1% non-fat skimmed milk dissolved in 1×TBST overnight at 4°C. The next day, the membranes were washed three times with 1×TBST and incubated with secondary antibody (Anti-mouse IgG-HRP-linked Antibody, Cell Sigaling Technology #7076) diluted 1:5000 in 1% non-fat skimmed milk dissolved in 1×TBST for 1 hour at room temperature. The membrane was again washed three times with 1×TBST. Lastly, an enhanced luminol-based chemiluminescent (ECL) substrate, SuperSignal^{™} West Pico PLUS Chemiluminescent Substrate (Thermo Scientific #34577) was added to the membrane and the signal development was documented with Bio-rad Gel Doc MP Imaging System. Rabbit anti-β-Actin-HRP linked Antibody (Cell Signaling Technology #5125) was used as an even protein loading control.

Westem-blot transfer buffer: Contains 25 mM Tris, 192 mM glycine, pH 8.3, with 20% fresh methanol (vol/vol). For the transfer of large proteins (> 2000 kDa, 0.1% SDS is added).

1× TBST for 1000 ml: 20mM Tris-base 2.4 Gram, 150mM NaCl 8.76 Gram, 50mM KCl 3.73 Gram, 0.2% Tween-20 (Tween^{®} 20, Carl Roth^{®} #9127.1), adusted pH to 7.6 and add ddH2O to 1000ml.

### 14) Testing the expression of Ras protein on the cell surface with flow cytometry.

Two hundred thousand cells were used for each flow cytometry sample. For adherent cell lines, cell culture medium was first removed and the cells were washed one time with PBS. After washing, 1 ml of 0.5% Trypsin-EDTA (Gibco, #25300054) was added to trypsinise the cells for 3 min at 37°C. Then, 9 ml of complete medium (Table 1) was added to stop trypsinisation process. Suspension cells were directly taken from the cell culture medium. Cell number of each sample was determined via Trypan Blue staining and a total of 2 × 10⁶ cells were harvested and washed one time with ice-cold PBS. The cells were then resuspended into 1 ml of ice-cold FACS buffer (PBS supplemented with 2% FBS) and blocked with 10 µl of human FcR Blocking Reagent (MACS Miltenyi #130-059-901) for 10 min at 4°C. The cells were then aliquoted into a 96-well round bottom plate with 100 µl per well. One microliter of primary antibodies namely PCC_X1_01 (Pan Ras Monoclonal Antibody (Ras10), Invitrogen #MA1-012) and PCC_X1_02 (Ras (27H5) Rabbit mAb, Cell Signalling Technology #3339) was added into their respective sample wells and incubated for 1 hour at 4°C in dark. Then, cells were pelleted through centrifugation at 1500 rpm for 5 min at 4°c and washed with 100 µl of FACS buffer for 3 times. After last wash, cells were resuspended into 100 µl FACS buffer and 1 µl of the appropriate secondary antibodies (FITC anti-mouse IgG (Biolegend #406001) for PCC_X1_01; and FITC anti-rabbit IgG (Biolegend #406403) for PCC_X1_02) was added into their respective wells and incubated for 30 min at 4°C in dark. The cells were washed 3 times with FACS buffer after secondary antibody incubation and finally resuspended into 100 µl FACS buffer and 1 µl of 7-AAD Viability Staining Solution (Biolegend #420404), to exclude dead cells, was added to all sample wells. After 5 minutes, flow cytometry analysis was performed with BD FACSCanto II Flow Cytometry System. Single staining controls were performed to correct spectral spillover.

### 15) Testing the expression of Ras protein on the cell surface of different tumour cells under normal and stress-conditions with an Enzyme-linked Immunosorbent Assay (ELISA).

For the cell-based ELISA, different adherent cell lines (AsPC-1, Panc-1, SK-BR-3 and BT-474) were used. First, the cell culture medium was removed, and the cells were washed one time with PBS. After washing, 2 ml of 0.5% Trypsin-EDTA (Gibco, #25300054) was added to trypsinise the cells for 3 min at 37°C. Then, 13 ml of complete medium (Table 1) was added to stop the trypsinisation process. Cell numbers were count via Trypan Blue staining and 30.000 viable cells in 50 µl were seeded out per well, in a 96-well tissue culture plate at 37°C and 5% CO2. After overnight adhesion of the cells, three different mechanisms to induce cellular stress on the tumour cells were tested: (i) addition of 0,5% DMSO in the cell culture medium, (ii) adding 1,5 µM Lenalidomide in the cell culture medium, (iii) or replacement of the complete medium by cell culture medium without supplemented FBS for serum-starvation. As normal control, for cells not under additional cellular stress, cells were left in complete medium. The stress-induction was performed overnight at 37°C and 5% CO2. The next day, to reduce unspecific binding of detection antibody to the plastic of the cell culture plate, each well was filled up to the top with 50% FBS in PBS, carefully pipette on top of the incubation medium, and incubated for 30 minutes at 37°C. Then, the all medium was discarded from the wells and 50 µl of 1:100-diluted FcR Blocking Reagent (MACS Miltenyi #130-059-901) was added to each well to avoid unspecific binding by blocking of the Fc-Receptors on the tumour cells. After 15 minute incubation, 50 µl of the primary antibodies were added into their respective wells, on top of the blocking solution. Two different primary antibodies to detect Ras protein were used, the PCC_X1_01 (Pan Ras Monoclonal Antibody (Ras10), Invitrogen #MA1-012) and PCC_X1_02 (Ras (27H5) Rabbit mAb, Cell Signalling Technology #3339), and to control for unspecific detection of intra cellular proteins, two different antibodies against the intracellular proteins anti-Vinculin (Sigma-Aldrich, #V9131) and anti β-Tubulin (Invitrogen #MA5-16308) were used. As a positive control for an extracellular membrane protein expressed on the cell surface of the tumour cells an anti-EpCAM antibody (Cell Signaling Technology #2929S) was used. The antibodies were incubated for an hour at room temperature. This was followed by the washing of the plates three times with PBS-Tween 20 (0,05%) (Tween^{®} 20, Carl Roth^{®} article number: #9127.1). For detection, 50 µl of a 1:5000 dilution of the appropriate Horseradish peroxidase (HRP) labelled secondary antibody in PBS was used per well (IgG Mouse (Cell Signaling Technology, #7076) for PCC_X1.01, Vinculin, Tubulin and EpCAM and IgG Rabbit (Cell Signaling Technology, #7074) for PCC_X1.02). After 30 minutes of incubation at 37°C, the plates were washed again with PBS-Tween 20 (0,05%) three times to remove unbound HRP-detection Antibody. For measuring the bound HRP-activity, 100 µl of the ELISA-Substrate (Sigma-Aldrich #T4444) was added to each well.

To compensate for unspecific binding of the secondary HRP-labelled antibody to the tumour cells and/or the plastic of the cell culture plates under the different conditions (stress-induced, normal, positive and negative controls), an exact duplicate was run for each sample and plate, only without the addition of the primary antibody (PCC_X1.01, PCC_X1.02, anti-Vinculin, anti β-Tubulin or anti-EpCAM).

The HRP-reaction was stopped after sufficient colour development by adding 50 µl of stop solution (1 N Sulfuric Acid) to each well. Finally, the absorbance was measured in each well at a wavelength of 450 nm and a reference wavelength of 630 nm with a Tecan SPARK^{®} multimode-mikroplate-reader. The absorbance values from the unspecific HRP-labelled control samples (samples without primary antibody but with corresponding HRP labelled secondary antibody), were used for the zero baseline model and set at 0 (Figure 19 and 20).

### REFERENCES

The references are:
[1] Wang, et al. "Identification of T-cell Receptors Targeting KRAS-Mutated Human Tumors." Cancer immunology research vol. 4,3 (2016): 204-14. doi:10.1158/2326-6066.CIR-15-0188.
[2] Douglass J, et al. "Bispecific antibodies targeting mutant RAS neoantigens". Sci Immunol. 2021 Mar 1;6(57):eabd5515.
[3] Heider, et al. "The IMiD target CRBN determines HSP90 activity toward transmembrane proteins essential in multiple myeloma." Mol Cell. 2021 Mar 18;81(6): 1170-1186.e10.
[4] Teo MYM, et al. "Development of a single-chain fragment variable fused-mutant HALT-1 recombinant immunotoxin against G12V mutated KRAS colorectal cancer cells". PeerJ. 2021 Apr 15;9:e11063.
[5] Crystal Structure ofMb(NS1)/H-Ras Complex. PDB DOI: https://doi.org/10.2210/pdb5E95/pdb.
[6] Spencer-Smith R, et al. "Inhibition of RAS function through targeting an allosteric regulatory site". Nat Chem Biol. 2017 Jan;13(1):62-68.
[7] Brischwein K, et al.. "MT110: a novel bispecific single-chain antibody construct with high efficacy in eradicating established tumors". Mol Immunol. 2006 Mar;43(8): 1129-43. doi: 10.1016/j.molimm.2005.07.034. Epub 2005 Sep 1. PMID: 16139892.
[8] Rodrigues ML, et al. "Engineering a humanized bispecific F(ab')2 fragment for improved binding to T cells". Int J Cancer Suppl. 1992;7:45-50. PMID: 1428403.
[9] Waters AM, et al. "Evaluation of the selectivity and sensitivity of isoform- and mutation-specific RAS antibodies". Sci Signal. 2017 Sep 26;10(498):eaao3332.
[10] Blair HA. "Sotorasib: First Approval. Drugs". 2021 Sep;81(13): 1573-1579. doi: 10.1007/s40265-021-01574-2. Erratum in: Drugs. 2021 Nov;81(16): 1947. PMID: 34357500; PMCID: PMC8531079.

## Claims

1. An Antigen Binding Protein (ABP), wherein the ABP comprises at least a first antigen binding domain capable of binding to an extracellular Ras antigen, provided that said extracellular Ras antigen is not part of a human leukocyte antigen (HLA)-peptide complex presented on the cell surface by the major histocompatibility complex (MHC), and wherein said ABP optionally comprises one or more additional antigen binding domain(s) that is capable of binding to antigen(s) present on a mammalian T-cell, preferably a human cluster of differentiation 3 (CD3) antigen or a human T cell receptor (TCR), optionally wherein said ABP enhances a cell-mediated immune response, such as the immune response mediated by an activated cytotoxic T-cell (CTL) to a mammalian cell expressing said extracellular Ras antigen.

2. The ABP according to claim 1, wherein the extracellular Ras antigen comprises, preferably consists of, the sequence according to any one of SEQ ID NOs: 1, 3, 5, 7, or 9, or comprises, preferably consists of, a sequence having at least 75% sequence identity, preferably at least 80%, more preferably at least 90%, and most preferably at least 95% sequence identity with any one of SEQ ID NOs: 1, 3, 5, 7, or 9, optionally wherein the extracellular Ras antigen is a mutant Ras antigen comprising, preferably consisting of, a sequence having at least 75% sequence identity, preferably at least 80%, more preferably at least 90%, and most preferably at least 95% sequence identity with a sequence according to any one of SEQ ID NOs: 11 to 19, or 77 to 119.

3. The ABP according to claim 1 or 2, wherein said ABP is selected from the group consisting of an immunoglobulin molecule, such as an IgG, IgE, IgD, IgA, or IgM immunoglobulin, preferably an IgG immunoglobulin, a monoclonal antibody, a chimeric antibody, a bispecifc ABP, such as a bispecific antibody, a CDR-grafted antibody, a humanized antibody, a single domain antibody, such as a V_{H}H single domain antibody, a hemibody antibody, a single-chain KeyLock-antibody, a diabody, a single chain diabody, a variable domain of the antibody heavy chain or antibody light chain, a multispecific antibody, a Chimeric Antigen Receptor (CAR), alternative protein binders including monobodies (derived from fibronectin type III), anticalins (derived from lipocalins), affibodies (derived from immunoglobulin-binding protein A), DARPins (Designed Ankyrin Repeat Proteins), proteins with repeating motifs like leucine-rich repeats (LRRs), ankyrin repeats (ARs), Armadillo repeats (Arms), tetratricopeptide repeats (TPRs), and/or a fragment of an antibody, such as a fragment of a monoclonal antibody, for example a single chain Fv (scFv), (scFv)₂, a Fv, a disulfide linked Fv, Fab, Fab', F(ab')₂ or a scFv-Fc, preferably wherein said ABP is a bispecific antibody or a CAR.

4. The ABP according to any one of claims 1 to 3, wherein the ABP is a bispecific antibody, and comprises at least one additional antigen binding domain that binds to a human cluster of differentiation 3 (CD3) antigen, preferably wherein the antigen binding domain that binds to a human cluster of differentiation 3 (CD3) antigen comprises, preferably consists of, a sequence having at least 75% sequence identity, preferably at least 80%, more preferably at least 90%, and most preferably at least 95% sequence identity with an amino acid sequence according to SEQ ID No: 48 or 49.

5. The ABP according to any one of claims 1 to 4, comprising:
(i) a heavy chain variable domain comprising the CDRH1 region set forth in SEQ ID NO: 50 and/or 53, the CDRH2 region set forth in SEQ ID NO: 51 and/or 54, and the CDRH3 region set forth in SEQ ID NO: 52 and/or 55, or wherein in each case independently the CDRH1, CDRH2 and/or CDRH3 comprise a sequence having no more than three or two, preferably no more than one amino acid substitution(s), deletion(s) or insertion(s) compared to SEQ ID NO: 50 and/or 53, SEQ ID NO: 51 and/or 54, or SEQ ID NO: 52 and/or 55, respectively; or comprising a CDRH1, CDRH2 or CDRH3 sequence having at least 75% sequence identity, preferably at least 80% sequence identity, more preferably at least 90% sequence identity, and most preferably at last 95% sequence identity with SEQ ID NO: 50 and/or 53, SEQ ID NO: 51 and/or 54, or SEQ ID NO: 52 and/or 55; and
(ii) a light chain variable domain comprising the CDRL1 region set forth in SEQ ID NO:56 and/or 59, the CDRL2 region set forth in SEQ ID NO: 57 and/or 60, and the CDRL3 region set forth in SEQ ID NO: 58 and/or 61, or wherein in each case independently CDRL1, CDRL2 and/or CDRL3 comprise a sequence having no more than three or two, preferably no more than one amino acid substitution(s), deletion(s) or insertion(s) compared to SEQ ID NO: 56 and/or 59, SEQ ID NO: 57 and/or 60, or SEQ ID NO: 58 and/or 61, respectively; or comprising a CDRL1, CDRL2 or CDRL3 sequence having at least 75% sequence identity, preferably at least 80% sequence identity, more preferably at least 90% sequence identity, and most preferably at last 95% sequence identity with SEQ ID NO: 56 and/or 59, SEQ ID NO: 57 and/or 60, or SEQ ID NO: 58 and/or 61.

6. The ABP according to any one of claims 1 to 5, wherein the heavy chain variable region comprises an amino acid sequence having a sequence identity of at least 70%, preferably at least 80%, more preferably at least 90%, and most preferably at last 95% sequence identity, to an amino acid sequence selected from SEQ ID NO: 62 and/or 64, or, in each case independently, optionally with no more than ten, nine, eight, seven, six, five, four, preferably no more than three, two or one, amino acid substitution(s), insertion(s) or deletion(s) compared to these sequences; and/or wherein the light chain variable region comprises the amino acid sequence having a sequence identity of at least 70%, preferably at least 80%, more preferably at least 90%, and most preferably at last 95% sequence identity, to the amino acid sequence selected from SEQ ID NO: 63 and/or 65, or, in each case independently, optionally with no more than ten, nine, eight, seven, six, five, four, preferably no more than three, two or one, amino acid substitution(s), insertion(s) or deletion(s) compared to these sequences.

7. The ABP according to any one of claims 1 to 6, wherein the heavy chain variable region comprises an amino acid sequence having a sequence identity of at least 70%, preferably at least 80%, more preferably at least 90%, and most preferably at last 95% sequence identity, to an amino acid sequence selected from SEQ ID NO: 66, 68, and 70, or, in each case independently, optionally with no more than ten, nine, eight, seven, six, five, four, preferably no more than three, two or one, amino acid substitution(s), insertion(s) or deletion(s) compared to these sequences; and/or wherein the light chain variable region comprises the amino acid sequence having a sequence identity of at least 70%, preferably at least 80%, more preferably at least 90%, and most preferably at last 95% sequence identity, to the amino acid sequence selected from SEQ ID NO: 67, 69, and 71, or, in each case independently, optionally with no more than ten, nine, eight, seven, six, five, four, preferably no more than three, two or one, amino acid substitution(s), insertion(s) or deletion(s) compared to these sequences.

8. The ABP according to any one of claims 1 to 7, wherein the ABP is a heterodimer comprising two different heavy chain immunoglobulin constant domains, wherein the first heavy chain comprises a first mutation (such as a knop mutation), and the pairing second heavy chain comprises a corresponding second mutation (such as a hole mutation), wherein the first mutation and the second mutation allow to form a heterodimer, optionally wherein the first heavy chain comprises an amino acid sequence according to SEQ ID No: 42 and the second heavy chain comprises an amino acid sequence according to SEQ ID No: 43.

9. A Chimeric Antigen Receptor (CAR), comprising from N-terminus to C-terminus:
(a) an extracellular domain comprising at least a first antigen binding domain capable of binding to an extracellular Ras antigen, provided that said extracellular Ras antigen is not part of a HLA-peptide complex presented on the cell surface by MHC, preferably wherein the antigen binding domain comprises the amino acid sequence having a sequence identity of at least 70%, more preferably at least 80%, even more preferably at least 90%, and most preferably at least 95% sequence identity, to an amino acid sequence selected from SEQ ID NO: 20, 21, 22, 23, 24, 25, 26, 27, 28, 29, 30, 31, 66, 67, 68, 69, 70, or 71 or, in each case independently, optionally with no more than ten, nine, eight, seven, six, five, four, preferably no more than three, two or one, amino acid substitution(s), insertion(s) or deletion(s) compared to these sequences;
(b) an extracellular hinge domain,
(c) a transmembrane domain, and
(d) a cytoplasmic domain.

10. An isolated nucleic acid comprising a sequence encoding for an ABP according to any one of claims 1 to 8, or encoding for a CAR according to claim 9, or an expression construct for expressing the ABP according to any one of claims 1 to 8, or a CAR according to claim 9, in a (host) cell, preferably further comprising promoter and/or terminator sequences.

11. A recombinant host cell expressing an ABP according to any one of claims 1 to 8 or a CAR according to claim 9, or comprising an isolated nucleic acid according to claim 10, or an expression construct according to claim 10, wherein the recombinant host cell is preferably selected from effector cells of the immune system, such as lymphocytes, for example CD8 positive cytotoxic lymphocytes, CD4 positive T cells, T helper cells, or Th17 T cells, natural killer (NK) cells, natural killer T (NKT) cells, dendritic cells, killer dendritic cells, B cells, γδ T cells, and a lymphocyte preparation containing NK cells and NKT cells mast cells.

12. A pharmaceutical composition comprising: (i) an ABP according to any one of claims 1 to 8, or (ii) a CAR according to claim 9, or (iii) an isolated nucleic acid according to claim 10, or (iv) an expression construct according to claim 10, or (v) a recombinant host cell according to claim 11, and a pharmaceutically acceptable carrier, stabilizer and/or excipient.

13. A pharmaceutical combination comprising:
(i) an ABP according to any one of claims 1 to 8, a CAR according to claim 9, an isolated nucleic acid according to claim 10, an expression construct according to claim 10, a recombinant host cell according to claim 11, or a pharmaceutical composition according to claim 12, and
(ii) a chemical compound, wherein the chemical compound is an organic solvent or an inhibitor increasing the expression of a Ras protein on the extracellular site of the plasma membrane, preferably wherein the chemical compound is a cereblon (CRBN) inhibitor, or a polar organic solvent like Dimethylsulfoxid (DMSO).

14. A compound for use in medicine, wherein the compound is selected from an ABP according to any one of claims 1 to 8, a CAR according to claim 9, an isolated nucleic acid according to claim 10, an expression construct according to claim 10, a recombinant host cell according to claim 11, a pharmaceutical composition according to claim 12, and a pharmaceutical combination according to claim 13.

15. A compound for use in the diagnosis, prevention and/or treatment of a proliferative disorder, such as cancer, and/or for use in adoptive, target-cell specific immunotherapy, wherein the compound is selected from an ABP according to any one of claims 1 to 8, a CAR according to claim 9, an isolated nucleic acid according to claim 10, an expression construct according to claim 10, a recombinant host cell according to claim 11, a pharmaceutical composition according to claim 12, and a pharmaceutical combination according to claim 13, wherein the cancer is preferably selected from one or more of brain tumor, oesophageal cancer, mouth cancer, tongue cancer, thyroid cancer, lung cancer, stomach cancer, pancreatic cancer, liver cancer, bile duct cancer, kidney cancer, colon cancer, rectal cancer, prostate cancer, bladder cancer, cervical cancer, epithelial cell cancers, skin cancer, leukaemia, lymphoma, myeloma, myeloid malignancies, lymphoid malignancies, breast cancer, ovarian cancer, neuroendocrine carcinoma, endometrial cancer, vaginal cancers, blood-related cancers, uterine cancer, testicular cancer, glioma, bone sarcoma, cervix cancer, Synovial cancer, and sarcoma, more preferably wherein the cancer is neuroendocrine carcinoma, for example high-grade poorly differentiated neuroendocrine carcinoma (NEC-G3), optionally wherein the cancer is metastatic, stage III cancer, or stage IV cancer, optionally wherein the cancer is a Ras dependent cancer.

16. A combination of an ABP according to any one of claims 1 to 8, a CAR according to claim 9, an isolated nucleic acid according to claim 10, an expression construct according to claim 10, a recombinant host cell according to claim 11, and a pharmaceutical composition according to claim 12, and a chemical compound, wherein the chemical compound is an organic solvent or an inhibitor increasing the expression of Ras protein on the extracellular site of the plasma membrane, preferably wherein the chemical compound is a cereblon (CRBN) inhibitor, or a polar organic solvent like DMSO, for use in a method of diagnosis, prevention and/or treatment of a proliferative disorder, such as cancer.
